# EUROPÄISCHE PATENTSCHRIFT

(11) **EP 3 658 066 B1**
(45) Veröffentlichungstag und Bekanntmachung des Hinweises auf die Patenterteilung: **14.04.2021**
(21) Anmeldenummer: 19746077.7
(22) Anmeldetag: 26.07.2019
(51) Int. Cl.: A61C 5/66, A61C 5/62, A61C 9/00

(54) **VORRICHTUNG ZUR AUFNAHME UND APPLIKATION VON DENTALMATERIAL UND VERFAHREN**
DEVICE FOR RECEIVING AND APPLYING DENTAL MATERIAL AND METHOD
DISPOSITIF DE RÉCEPTION ET D'APPLICATION DE MATÉRIAU DENTAIRE ET PROCÉDÉ

(30) Priorität: 31.07.2018 DE 102018118577
(43) Veröffentlichungstag der Anmeldung: 03.06.2020
(73) Patentinhaber: VOCO GmbH, 27472 Cuxhaven (DE)
(72) Erfinder: FONTEIN, Nils, 27472 Cuxhaven (DE); PLAUMANN, Manfred Thomas, 27472 Cuxhaven (DE); LEINER, Uwe, 27476 Cuxhaven (DE)
(74) Vertreter: Eisenführ Speiser
(86) Internationale Anmeldenummer: PCT/EP2019/070215
(87) Internationale Veröffentlichungsnummer: WO 2020/025485

(56) Entgegenhaltungen:
- WO-A1-2009/106352
- US-A- 5 122 057
- US-A1- 2006 204 924
- US-A1- 2015 297 325

## Beschreibung

Die vorliegende Erfindung betrifft das Gebiet der Handhabung von Materialien und insbesondere die Aufnahme von Dentalmaterial in einer Vorrichtung und die Applikation des Dentalmaterials aus einer solchen Vorrichtung, sowie ein Verfahren zum Behandeln von Dentalmaterial.

US 2015/0297325 A1 offenbart eine Mischvorrichtung zum Mischen einer dentalen Zusammensetzung mit einem Mischvorrichtungsgehäuse, wobei eine Wand des Mischvorrichtungsgehäuses einen Lichtfilter bildet. Der Lichtfilter sorgt für ein substantielles Blockieren von Licht bestimmter Wellenlängen, wobei er Licht zumindest anderer Wellenlängen durchlässt. Die Lehre von US 2015/0297325 A1 kann dabei helfen, dentale Zusammensetzungen gegenüber einer Zersetzung zu schützen.

US 2006/0204924 A1 offenbart eine Kartusche zum Lagern und Ausgeben einer Masse, insbesondere eines Dentalmaterial, und umfasst einen röhrenförmigen Basiskörper mit einem ersten zylindrischen Abschnitt und einem zweiten gebogenen Abschnitt und einer Extrusionstülle. Ein Lastaufnahmeelement kooperiert mit einer Extrusionsantriebsvorrichtung, die aktiviert werden kann, um eine Extrusionsoperation auszuführen, in der die Masse in der Kartusche aus der Extrusionstülle extrudiert wird, Die Kartusche umfasst ferner einer axial beweglichen Kolben, der in dem Basiskörper angeordnet ist und dazu ausgestaltet ist, durch die Operation der Extrusionsantriebsvorrichtung bewegt zu werden, so dass der Kolben, wenn er sich relativ innerhalb des ersten zylindrischen Abschnitts und des zweiten gebogenen Abschnitts des Basiskörpers bewegt, die Masse aus dem Basiskörper in die Extrusionstülle und darüber hinaus drückt. Der innere Durchmesser der Extrusionstülle ist geringer als der innere Durchmesser des Basiskörpers und der Kolben ist zumindest teilweise biegsam.

US 5,122,057 offenbart eine dentale Kartusche zur Abgabe eines Dentalmaterial mit einem Körperabschnitt, der aus einem Material hergestellt ist, das für wenigstens einen Teil des sichtbaren Lichtspektrums transparent und für Licht opak ist, das hinsichtlich des darin enthaltenen Dentalmaterials photochemisch aktiv ist. US 5,122,057 offenbart ferner eine dentale Kartusche mit einem Körperabschnitt, der aus einem toroidalen Segment und einer daran angebrachten koaxialen Düse besteht. Die dentalen Kartuschen werden in einer Dentalspritze zur Applikation des Dentalmaterials an einem Patienten verwendet. WO 2009/106352 A1 offenbart thermochrom beschichtete Substrate und Verfahren zu deren Herstellung. Ein organischer thermochromer Komplex, bestehend aus mindestens einem Farbstoff, mindestens einem Entwickler und mindestens einem Schmelzmittel, wird im Vakuumprozess auf ein Substrat abgeschieden. Das Substrat kann aus Glas, Keramik, Metall und deren Komplexe bzw. Oxide, dotiertes und undotiertes Halbleitermaterial oder Kunststoff bestehen. Die Kombination unterschiedlicher thermochromer Schichten ist möglich. Die thermochrome Schicht kann mit weiteren funktionellen Schichten kombiniert werden, wobei diese ebenfalls bedampft werden, oder mittels Sputtern oder CVD/OVPD-Technik abgeschieden werden oder durch Siebdruck, Sprühen, Schleudern bzw. gängigen Technologieverfahren aufgetragen werden. Der thermochrome Effekt ist reversibel, wobei der Schaltbereich vorher bestimmt werden kann.

Als ein Beispiel einer Vorrichtung zur Aufnahme und Applikation eines Dentalmaterials kann eine sogenannte Compule betrachtet werden.

Compulen sind als Packmittel für Dentalmassen aus dem Stand der Technik hinlänglich bekannt (EP 1 689 314 A1, EP 2 364 123 A1, EP 2 696 794 A1, EP 0 848 935 A1, EP 2 412 448 A2, EP 0 669 113 B1, DE 102 18 859 A1, DE 10 2013 004 077 A1, US 6,877,983 B1). Sie ermöglichen es für jeden Patienten eine vorproportionierte Menge Füllungsmaterial zu lagern, so dass für jeden einzelnen Patienten jeweils ein neues, frisches Material verwendet wird und Querkontamination effektiv ausgeschlossen werden kann. Sie ermöglichen so einen hygienischen Arbeitsablauf.

Um die Dentalmaterialien zu verflüssigen, um sie einfacher ausdrückbar zu machen und ein besseres Anfließverhalten in der Kavität zu ermöglichen, wurden unterschiedliche Lösungen zu ihrer Erwärmung vorgeschlagen.

US 7,097,452 B2 beschreibt eine Compule zum Lagern und Erwärmen von dentalen Kompositen, deren Körper aus einem elektrisch leitenden und einem wärmeleitenden Kunststoff besteht. Zum Erwärmen wird eine externe Stromquelle benötigt.

EP 1 740 119 A1 beschreibt eine Compule zum Lagern und Ausbringen von dentalen Kompositen, deren Körper eine Energieabgabevorrichtung enthält. Diese Energieabgabevorrichtung ist z.B. ein spiralförmig angeordneter Heizdraht zum Erwärmen der Compule und deren Inhalt.

US 7,086,861 B2 offenbart eine Compule zum Lagern und Ausbringen von dentalen Kompositen, deren Körper eine elektrisch betriebene Induktionsspule aufweist, die dazu dient, die Compule und damit ihren Inhalt zu erwärmen.

Neben diesen speziellen, heizbaren Compulen können auch die Dispenser so ausgestaltet sein, dass sie das Erwärmen der Compulen samt deren Inhalt ermöglichen.

EP 1 190 681 A2 offenbart einen Dispenser, der über eine elektrische Heizeinheit verfügt und so Standardcompulen samt deren Inhalt erwärmen kann.

DE 10 2016 106 019 A1 offenbart eine Dispenservorrichtung für Dentalmaterialien, die als Heizeinheit einen Schichtheizkörper aufweist, der die Compule samt deren Inhalt erwärmt.

WO 2016/014605 A1 offenbart eine selbstheizende Hülse, die entweder eine Compule vorheizen kann oder die so ausgestaltet ist, dass sie die Compule im Dispenser erwärmen kann.

Es wurde gefunden, dass bekannte Ansätze zur Erwärmung der Dentalmaterialien verschiedene Nachteile aufweisen. Insbesondere dann, wenn zunächst die Compule oder eine sonstige Vorrichtung erwärmt wird, die dann wiederum Wärme an das zu erwärmende Dentalmaterial abgibt, besteht ein Problem im Wärmedurchgang durch die Compule oder Vorrichtung und im Wärmeübergang von der Compule bzw. Vorrichtung in das Dentalmaterial, wobei zudem im Dentalmaterial auch ein Wärmegradient entsteht, so dass eine gewünschte Erhöhung der Fließfähigkeit des Dentalmaterials nicht in gleichmäßiger Form erreicht werden kann. Zudem kann es bei einem längeren Heizvorgang vorkommen, dass die Temperatur des Compulenmaterials oder auch des in der Nähe der Wandung vorliegenden Dentalmaterials zu hoch wird, zumindest soweit nicht entsprechende Gegenmaßnahmen wie eine Begrenzung der Heiztemperatur vorgenommen werden, die sich aber wiederum negativ auf den Wärmeeintrag auswirkt.

Ein der vorliegenden Erfindung zugrundeliegendes Ziel ist es, eine Vorrichtung zur Aufnahme und Applikation eines Dentalmaterials und ein Verfahren zur Behandlung eines Dentalmaterials bereitzustellen, die die Nachteile des Standes der Technik vermeiden oder nur in geringerem Maße teilen.

Es ist daher gewünscht, eine Lösung vorzustellen, die eine einfache und praxisgerechte Handhabung eines Dentalmaterials im Sinne einer Aufnahme, Applikation und/oder Behandlung desselben erlaubt.

Erfindungsgemäß wird nach einem ersten Aspekt eine Vorrichtung zur Aufnahme und Applikation von Dentalmaterial vorgeschlagen, wie sie in Anspruch 1 definiert ist, nämlich mit einem Hohlraum zur Aufnahme des Dentalmaterials und einer den Hohlraum umgebenden Wandung, wobei die Wandung in wenigstens einem ersten Temperaturbereich für Strahlung wenigstens einer ersten Wellenlänge oder eines ersten Wellenlängenbereichs im Bereich von 100 nm bis 500 nm undurchlässig ist, und wobei die Wandung wenigstens einen Bereich mit einem Trägermaterial, bevorzugt einem Polyamid-Material und/oder einem Polybutylenterephthalat-Material, und einem Filtermaterial, das außen am Trägermaterial, innen am Trägermaterial und/oder zwischen wenigstens zwei Schichten des Trägermaterials vorgesehen ist und/oder in das Trägermaterial eingebettet ist, aufweist, wobei das Filtermaterial ausgewählt ist aus der Gruppe bestehend aus den Perylenderivaten 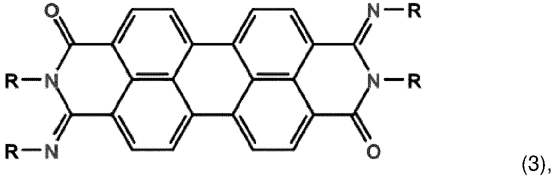 insbesondere 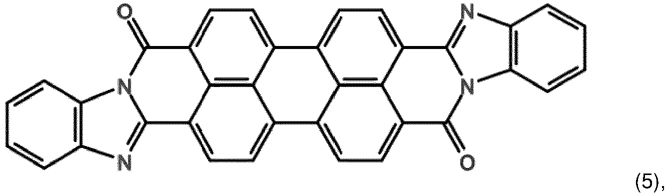 und 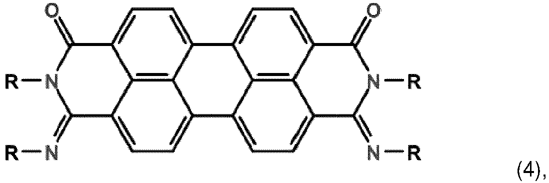 insbesondere 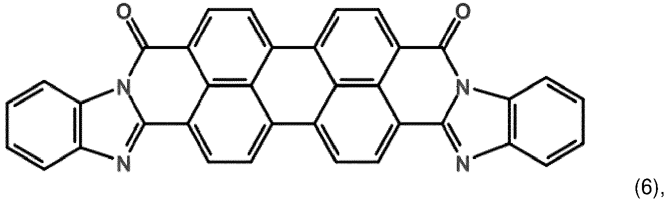 sowie Kombinationen davon.

Die Wandung der Vorrichtung kann auch einen Bereich aufweisen, der wenigstens in einem zweiten Temperaturbereich für Strahlung wenigstens einer zweiten Wellenlänge oder eines zweiten Wellenlängenbereichs im Bereich von 600 nm bis 50.000 nm durchlässig ist

Erfindungsgemäß wird nach einem zweiten Aspekt ein Verfahren zum Behandeln eines Dentalmaterials außerhalb des menschlichen oder tierischen Körpers, vorgeschlagen, wie es in Anspruch 10 definiert ist, nämlich mit den Schritten eines Bereitstellens einer erfindungsgemäßen Vorrichtung umfassend strahlungshärtbares Dentalmaterial in dem ersten Temperaturbereich, eines Bestrahlens der bereitgestellten Vorrichtung mit Strahlung der zweiten Wellenlänge oder des zweiten Wellenlängenbereichs, vorzugsweise mit IR-Licht einer Wellenlänge im Bereich von 600 nm bis 50.000 nm, zum Erwärmen des Dentalmaterials und eines Verringerns der Viskosität des Dentalmaterials durch die Erwärmung bis zu einer gewünschten Viskosität oder weniger.

Ein Teil des Hintergrunds der vorliegenden Erfindung findet sich in den folgenden Überlegungen.

Lichthärtende oder allgemeiner strahlungshärtbare Dentalmaterialien, also Dentalmaterialien, die zur Initiierung eines Härtungsprozesses und/oder zu dessen Unterstützung mit Licht einer bestimmten Wellenlänge oder eines bestimmten Wellenlängenbereichs bestrahlt werden, müssen vor der Anwendung gegenüber dieser Strahlung geschützt werden. Auch bei anderen Dentalmaterialien kann sich ein Interesse ergeben, einen unerwünschten Lichteinfall zu verhindern oder zumindest weitgehend zu reduzieren. Ein typischer Ansatz zur Verhinderung von Lichteinfall besteht darin, das Material der Verpackung (hier etwa das Compulenmaterial) mit Ruß ("carbon black") zu versetzen, so dass sich ein licht-undurchlässiges Material ergibt.

Eine besondere Empfindlichkeit im vorstehenden Sinne besteht bei Licht einer Wellenlänge im Bereich von 100 bis 500 nm.

Will man anstelle eines direkten Wärmeübergangs von einer Heizung an das Compulenmaterial oder eines Wärmeübergangs von der Heizung zur Luft und dann an das Compulenmaterial eine direkte Bestrahlung etwa einer Compule mit Infrarot-Strahlung vorsehen, so führt dies zwar zu einer Erwärmung des Compulenmaterials, das etwa mit carbon black versetzt ist, die Infrarot-Strahlung erreicht allerdings nicht das Dentalmaterial selbst, da die Infrarot-Strahlung vom Compulenmaterial bzw. dem darin enthaltenen Ruß/carbon black absorbiert wird.

Es wurde nun realisiert, dass es möglich ist, bei einer Vorrichtung zur Aufnahme und Applikation eines Dentalmaterials einen Schutz für das Dentalmaterial gegenüber unerwünschten Lichtdurchtritt mit einer Durchlässigkeit für Infrarot-Strahlung zu kombinieren, so dass zwar unerwünschte Strahlung abgehalten wird, die heizende Infrarot-Strahlung aber direkt an bzw. in das Dentalmaterial gelangen kann.

Auch wenn im Grunde der gesamte Bereich der Infrarot-Strahlung im Rahmen der Erfindung genutzt werden kann, besteht ein besonderes Interesse an der Strahlung im nahen Infrarot-Bereich (750 bis 3.000 nm Wellenlänge).

Es ist bei einer Umsetzung der Erfindung zu beachten, dass der für den Durchtritt der Strahlung wenigstens der zweiten Wellenlänge oder des zweiten Wellenlängenbereichs vorgesehene Wandungsbereich aufgrund des Materials der Wandung einen oder mehrere Absorptionsbereiche für eine oder mehrere Wellenlängen zwischen 600 und 50.000 nm aufweisen kann, wobei dann zu verstehen ist, dass die zur Erwärmung des Dentalmaterials verwendete Strahlung nicht nur lediglich in einem derartigen Absorptionsbereich vorliegen sollte. Wird beispielsweise eine Infrarot-LED mit einer vergleichsweise engen Emissionsbande zur Erwärmung des Dentalmaterials vorgesehen, sind die LED und die Wandung so zu wählen, dass Emission und Absorption nicht im Wesentlichen zusammenfallen.

Auch wenn ein relevanter Anteil der Anwendungsbereiche eines Verfahrens, bedingt durch die Natur und den Zweck des Dentalmaterials, Verfahrensschritte am oder im menschlichen oder tierischen Körper vorsieht, sind vorliegend erfindungsgemäße Verfahren in Betracht zu ziehen, die als solche keine therapeutischen Aspekte aufweisen. Hier seien beispielsweise die Verwendung des Dentalmaterials für nicht-therapeutische Verfahren (z.B. als Füllungsmaterial, Unterfüllungsmaterial, Befestigungsmaterial oder Ausbesserungs- oder Versiegelungsmaterial für Materialien (etwa Stein), die mit einem Zahnmaterial im weitesten Sinne vergleichbar sind) oder auch für Test- oder Schulungszwecke genannt.

Im Zusammenhang der vorliegenden Erfindung ist der Begriff "undurchlässig" nicht nur beschränkt auf eine vollständige Undurchlässigkeit im Sinne eine 100%igen Absorption und/oder Reflektion zu verstehen, wobei "undurchlässig" einen Transmissionsgrad von weniger als 10%, bevorzugt von weniger als 5%, besonders bevorzugt von weniger als 1% umfasst. Im Rahmen der technischen Machbarkeit kann jedoch auch eine aus praktischer Sicht vollständige Undurchlässigkeit vorgesehen sein.

Ebenso soll mit dem Begriff "durchlässig" nicht nur eine vollständige Transmission ohne Streuung, Reflektion und/oder Absorption adressiert werden, wobei ein Transmissionsgrad von mehr als 50%, bevorzugt von mehr als 65%, besonders bevorzugt von mehr als 80% als "durchlässig" anzusehen ist. Soweit sich aus den Umständen nichts anderes ergibt, sollte klar sein, dass eine aus praktischer Sicht vollständige Durchlässigkeit im Allgemeinen besonders vorteilhaft ist. Soweit der ursprüngliche Energieeintrag (also die eingestrahlte Lichtmenge) als solcher nicht besonderen Beschränkungen unterworfen ist, können bei der Betrachtung des Begriffs "durchlässig" auch Reflektion und Streuung als solche gänzlich außen vorgelassen werden, so dass in diesem Sinne eine Durchlässigkeit dann gegeben ist, wenn der Transmissionsgrad lediglich größer als der Absoprtionsgrad ist, wobei hierbei ein Mindestverhältnis von Transmissionsgrad zu Absorptionsgrad von 3:1, besonders 5:1, insbesondere 10:1 bevorzugt ist.

Es wurde gefunden, dass mit der oben angegebenen Kombination eines Trägermaterials (insbesondere Polyamid) und eines Filtermaterials in Form eines oder mehrerer Perylenderivate unabhängig davon, ob ein Transmissionsgrad (unter Einbeziehung von Streuung, Reflektion und/oder Absorption) von mehr als 50% vorliegt, vorteilhafte Ergebnisse erzielt werden können. Da diese Kombination durchaus auch die Merkmale des Aspekts A) erfüllen kann, besteht eine gewisser Überlapp zwischen den Aspekten A) und B), wobei der Aspekt B) auch (als solche nicht unter Aspekt A) fallende) Vorrichtungen betrifft, bei denen die Wandung lediglich einen Bereich mit der angegebenen Kombination von Trägermaterial und Filtermaterial aufweist, ohne allerdings "durchlässig" im Sinne eines Transmissionsgrads (unter Einbeziehung von Streuung, Reflektion und/oder Absorption) von mehr als 50% zu sein. Es sollte klar sein, dass sich der Bereich mit dem Trägermaterial und dem Filtermaterial auch über die gesamte Wandung der Vorrichtung erstrecken kann. Hinsichtlich der relativen Menge des Filtermaterials zu Trägermaterial wird ein Anteil von 0,1 bis 8 Gew.-% bevorzugt, insbesondere 0,2 bis 5 Gew.-%, besonders bevorzugt 0,5 bis 2 Gew.-%.

In einer vorteilhaften Ausgestaltung eines Aspekts der Erfindung weist der Bereich der Wandung ein Filtermaterial auf oder besteht daraus, wobei das Filtermaterial zumindest im ersten Temperaturbereich, bevorzugt zumindest im ersten Temperaturbereich und im zweiten Temperaturbereich, für Strahlung der ersten Wellenlänge oder des ersten Wellenlängenbereichs einen Transmissionsgrad von weniger als 10%, bevorzugt von weniger als 5%, aufweist, wobei das Filtermaterial zumindest im zweiten Temperaturbereich für Strahlung der zweiten Wellenlänge oder des zweiten Wellenlängenbereichs einen Transmissionsgrad von mehr als 50%, bevorzugt von mehr als 65%, besonders bevorzugt von mehr als 80% aufweist.

Die Nutzung eines Filtermaterials mit einem geringen Transmissionsgrad für die erste Wellenlänge oder den ersten Wellenlängenbereich, der zumindest im ersten Temperaturbereich vorliegt, erlaubt einen wirksamen Schutz für das Dentalmaterial gegen die unerwünschte Einstrahlung. Es ist hierbei nicht unbedingt notwendig, wenn auch allerdings bevorzugt, dass das Filtermaterial zumindest auch im zweiten Temperaturbereich (besonderes bevorzugt auch im Bereich zwischen dem ersten und dem zweiten Temperaturbereich) einen solchen geringen Transmissionsgrad besitzt. Verliert das Filtermaterial mit steigender Temperatur seine Filterwirkung teilweise oder vollständig, kann dennoch bei einer entsprechend schnellen Applikation ein negativer Einfluss der Strahlung vermieden oder zumindest verringert werden.

Besitzt das Filtermaterial im Bereich des ersten und des zweiten Temperaturbereichs und ggf. dazwischen bereits eine hohe Durchlässigkeit für die zur Erwärmung des Dentalmaterials genutzte Strahlung der zweiten Wellenlänge oder des zweiten Wellenlängenbereichs, so kann diese Strahlung direkt an bzw. in das Dentalmaterial gelangen, um dieses zu erwärmen. Es wäre unschädlich, wenn das Filtermaterial etwa im ersten Temperaturbereich einen höheren Absorptionsgrad aufweist, soweit sich mit der Absorption verbundene Wärmeaufnahme zumindest im zweiten Temperaturbereich eine gewünschte Durchlässigkeit ergibt.

Vorzugsweise besitzt das Filtermaterial hinsichtlich seiner Durchlässigkeit für die Strahlung der jeweiligen Wellenlängen bzw. Wellenlängenbereiche keine oder eine allenfalls unwesentliche Temperaturabhängigkeit, wobei ein Filtermaterial mit einer merklichen Temperaturabhängigkeit, z.B. einer linearen Temperaturabhängigkeit im Gegensatz zu einer Temperaturabhängigkeit eines thermochromen Materials, nicht von der Erfindung ausgeschlossen ist, sofern die oben genannten Bedingungen erfüllt sind.

In einer bevorzugten Variante der obigen Ausgestaltung ist das Filtermaterial ausgewählt aus der Gruppe bestehend aus Substanzen mit einem Perylen-Gerüst, aromatische Diazoverbindungen und Kombinationen davon.

Im Rahmen der Erfindung sind als IR-transparente Farbpigmente Substanzen mit einem Perylen-Gerüst (Formel 1) als geeignet erkannt worden.

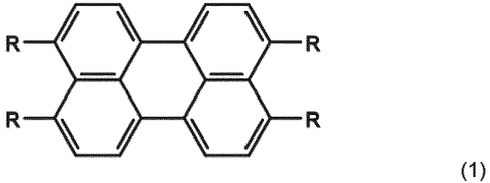

Es können beispielsweise die Perylentetracarbonsäure-Derivate der Formel 2 eingesetzt werden. Dabei steht X für O, NH oder NR, wobei R eine Alkylgruppe, eine Hydroxyalkylgruppe, eine aromatische oder eine substituierte aromatische Gruppe sein kann. Das N,N'-Bis(2-phenylethyl)perylene-3,4:9,10-bis(dicarboximid) (X = NCH₂CH₂Ph) ist beispielsweise kommerziell unter dem Namen Paliogen® Black S 0084 oder C.I. Pigment Black 31 erhältlich. Das N,N'-Bis(4-methoxybenzyl)perylene-3,4:9,10-bis(dicarboximid) (X = NCH₂(C₆H₄)OCH₃) ist beispielsweise kommerziell unter dem Namen Paliogen® Black L 0086 oder C.I. Pigment Black 32 erhältlich.

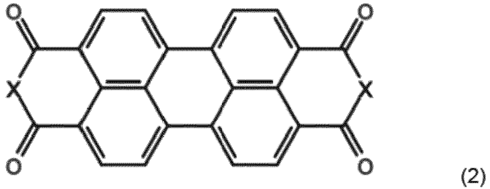

Als besonders geeignet gefunden wurden auch die Perylenderivate (3) bzw. (4), insbesondere wenn dabei die N-R-Gruppen Imidazole oder Pyrimidone, sowie annelierte Imidazole oder Pyrimidone bilden. Besonders bevorzugt sind die entsprechenden Perylenderivate (5) und (6). Solche Farbpigmente sind kommerziell als Lumogen® Black K0087 und Lumogen® Black K0088 erhältlich.

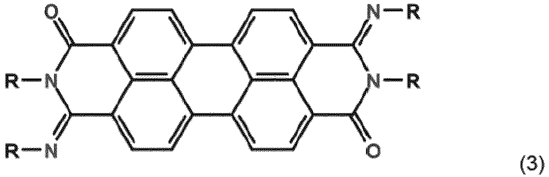

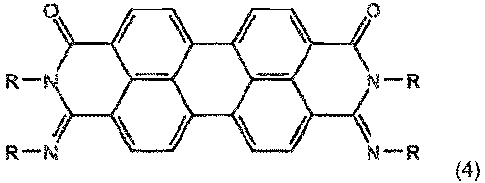

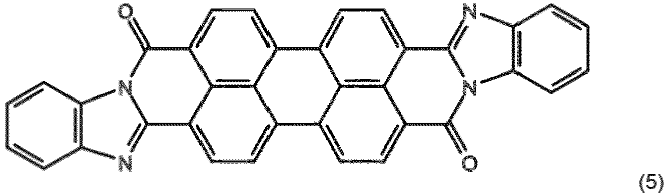

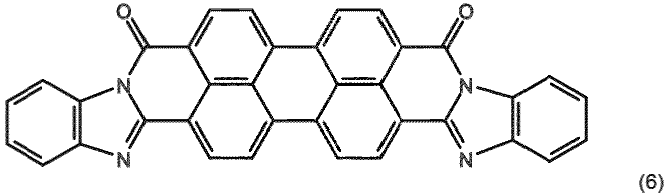

Weitere für die Erfindung geeignete IR-transparente Farbpigmente umfassen aromatische Diazoverbindungen der allgemeinen Formel 7.

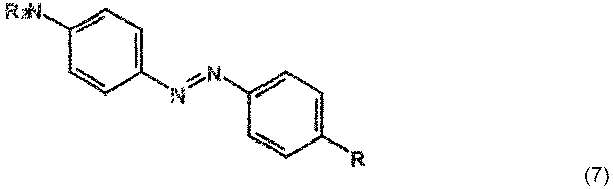

Beispiele sind das 4-(Dimethylamino)-4'-aminoazobenzol bzw. Diazo Black (8) oder Azomethine Black (9).

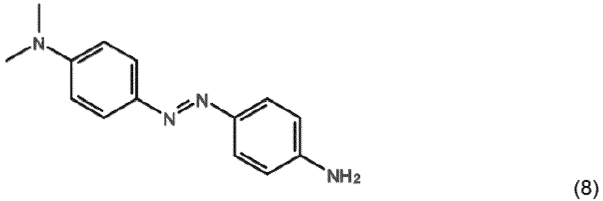

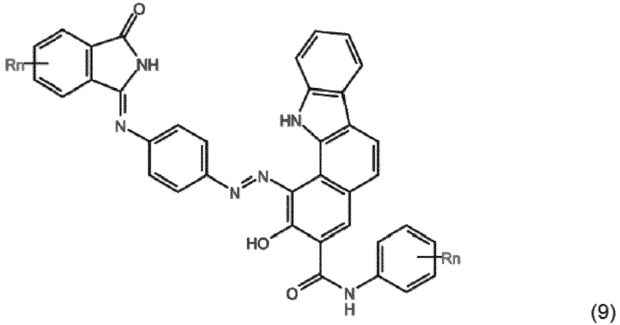

Die hier angegebenen Farbstoffe können als solche oder in mikroverkapselter Form eingesetzt werden. Vorteilhafterweise wird aus diesen Farbstoffen bzw. mikroverkapselten Farbstoffen zunächst ein Masterbatch bzw. ein Farbgranulat hergestellt, welches dann dem einzufärbenden Kunststoff zugegeben wird.

In einer anderen vorteilhaften Ausgestaltung eines Aspekts der Erfindung, die zur obigen Ausgestaltung alternativ oder ergänzend vorgesehen ist, weist der Bereich der Wandung thermochromes Material auf oder besteht daraus, das eine Schalttemperatur im Bereich von 25 bis 50°C besitzt, wobei das thermochrome Material bei einer Temperatur unter der Schalttemperatur für Strahlung wenigstens der zweiten Wellenlänge oder des zweiten Wellenlängenbereichs einen niedrigeren Transmissionsgrad besitzt als bei einer Temperatur über der Schalttemperatur.

Das thermochrome Material mit einer im niedrigen Temperaturbereich geringen Transmission für die Strahlung der zweiten Wellenlänge bzw. des zweiten Wellenlängenbereichs wird zunächst durch die Strahlung erwärmt, so dass aufgrund dieser Temperaturerwärmung schließlich die Schalttemperatur des thermochromen Materials erreicht oder überschritten wird, so dass das thermochrome Material für die Strahlung durchlässig(er) wird.

Auch hier ist es bei ausreichend kurzer Exposition (oder bei Vermeidung einer Umgebung, in der Strahlung der ersten Wellenlänge bzw. des ersten Wellenlängenbereichs vorliegt) unschädlich, wenn sich mit dem Umschalten des thermochromen Materials auch eine höhere Durchlässigkeit für diese Strahlung ergibt.

In einer bevorzugten Variante der obigen Ausgestaltung ist das thermochrome Material ausgewählt aus der Gruppe bestehend aus anorganischen Pigmenten umfassend Metallsalze oder Metalloxide, bei denen durch einen Phasenübergang, eine Änderung der Ligandengeometrie, eine Änderung der Koordinationszahl und/oder eine Änderung des Kristallfeldes ein Farbübergang stattfindet, organischen Pigmenten umfassend thermochrome Flüssigkristalle, konjugierte Polymere und Leukofarbstoffe und Kombinationen davon.

Als thermochrome Farbstoffe im Sinne eines Beispiels eines thermochromen Materials, das im Rahmen der vorliegenden Erfindung genutzt werden kann, können anorganische oder organische Pigmente eingesetzt werden.

Anorganische Pigmente sind Metallsalze oder Metalloxide, bei denen durch Phasenübergänge, Änderung der Ligandengeometrie, Änderung Koordinationszahl oder Änderung des Kristallfeldes ein Farbübergang stattfindet.

Organische Pigmente umfassen thermochrome Flüssigkristalle, konjugierte Polymere und Leukofarbstoffe. Thermochrome Flüssigkristalle sind beispielsweise Cholesterinderivate oder Cyanobiphenyle. Konjugierte Polymere können durch Konformationsänderungen Farbänderungen aufweisen. Als Leukofarbstoffe können Systeme auf Basis von Spiropyranen (Formel 11), Spirooxazinen (Formel 12), Salicyl-Schiff'schen Basen (Formel 13), Bianthronen (Formel 14), Indolylphthaliden (Formel 15) oder Fluoranen (Formel 16) eingesetzt werden.

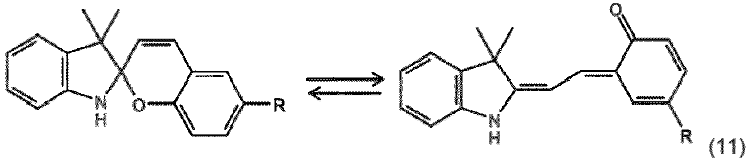

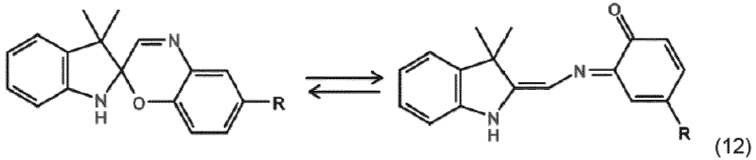

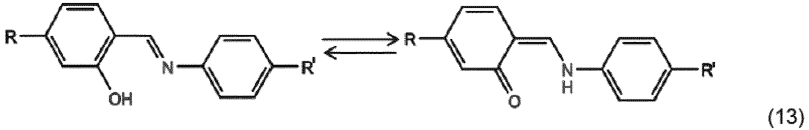

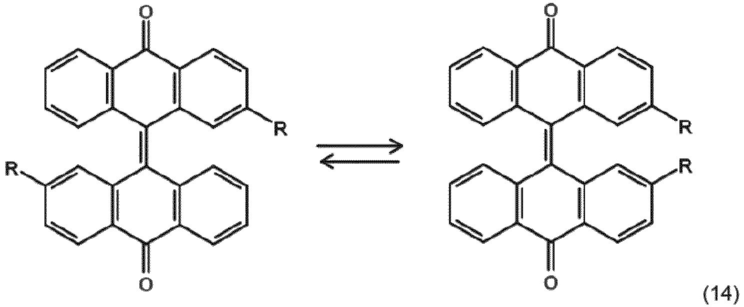

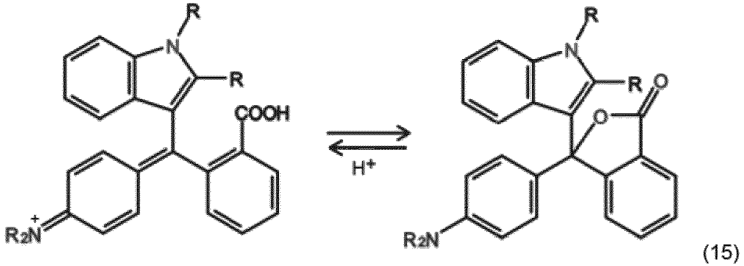

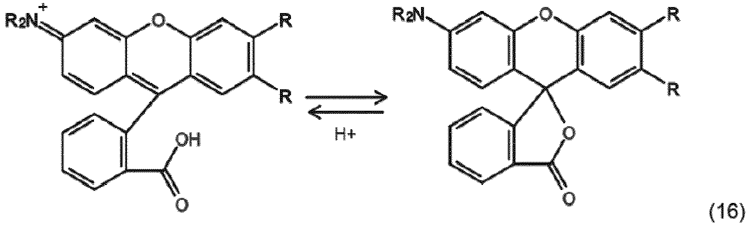

Solche Systeme bestehen in der Regel aus einem Elektronendonator (Spiropyrane, Spirooxazine, Salicyl-Schtff'sche Basen, Bianthrone, Indolylphthalide oder Fluorane) und einem Elektronenakzeptor. Bei den Elektronendonatoren lassen sich durch geeignete Wahl der Substituenten unterschiedliche Farben einstellen. Zusätzlich zu den in den Formeln vereinfacht dargestellten Positionen für die Substituenten sind auch andere Positionen denkbar. Als Elektronenakzeptoren werden Phenole, Azole oder organische Säuren eingesetzt. Beispielhafte Phenole sind Phenylphenol, Bisphenol A, Bisphenol AP, Bisphenol AF, Bisphenol FL, Cresol, Resorcinol, Chlorlucinol, Phenol, Phenololigomere, Naphthol, 1,5-Dihydroxynaphthalin, Pyrocatechol und Pyrogallol Beispielhafte Azole sind Benzotriazole, wie 5-Chlorbenzotriazol, 4-Laurylaminosulfobenzotriazol, 5-Butylbenzotriazol, Dibenzotriazol, 2-Oxybenzotriazol,5-Ethoxycarbonylbenzotriazol,5,5'-Methylenbisbenzotriazol, Imidazole, wie Oxybenzimidazol, und Tetrazole. Die organischen Säuren umfassen beispielsweise aromatische und aliphatische Carbonsäuren und substituierte Derivate davon. Beispiele für aromatische Carbonsäuren sind Salicylsäure, Methylenbissalicylsäure, Resorcylsäure, Gallussäure, Benzoesäure, p-Oxybenzoesäure, Pyromellitsäure, Naphthoesäure, Gerbsäure, Toluylsäure, Trimellithsäure, Phthalsäure, Terephthalsäure und Anthranilsäure. Beispiele für aliphatische Carbonsäuren sind Säuren mit 1 bis 20 Kohlenstoffatomen, vorzugsweise 3 bis 15 Kohlenstoffatomen, wie beispielsweise Stearinsäure, 1,2-Hydroxystearinsäure, Weinsäure, Zitronensäure, Oxalsäure und Laurinsäure.

Das thermochrome Material bzw. die thermochromen Materialien können als solche oder in mikroverkapselter Form eingesetzt werden. Vorteilhafterweise wird aus dem thermochromen Material oder dem mikroverkapselten thermochromen Material zunächst ein Masterbatch bzw. ein Farbgranulat hergestellt, welches dann dem einzufärbenden Kunststoff zugegeben wird.

In einer anderen vorteilhaften Ausgestaltung eines Aspekts der Erfindung weist die Wandung wenigstens ein Trägermaterial, bevorzugt ein Polyamid-Material und/oder ein Polybutylenterephthalat-Material, auf, wobei das Filtermaterial und/oder das thermochrome Material außen am Trägermaterial, innen am Trägermaterial und/oder zwischen wenigstens zwei Schichten des Trägermaterials vorgesehen ist und/oder in das Trägermaterial eingebettet ist.

Auch wenn im Rahmen der vorliegenden Erfindung bevorzugt Polyamid (PA) oder Polybutylenterephthalat (PBT) als Trägermaterial bzw. überhaupt als Material der Wandung eingesetzt werden, sind auch andere Kunststoffe für die erfindungsgemäße Vorrichtung geeignet, hierzu zählen die Standardthermoplaste Polyethylen (PE), Polypropylen (PP), Polystyrol (PS), Polyvinylchlorid (PCV) sowie Acrylnitril-Butadien-Styrol-Copolymer (ABS). Weiter geeignet sind die technischen Thermoplaste Polycarbonat (PC), Styrol-Acrylnitril-Copolymer (SAN), Polymethylmethacrylat (PMMA), Polyamid (PA), Polyoxymethylen (POM), Polybutylenterephthalat (PBT), Polypropylenterephthalat (PPT), Polyethylenterephthalat (PET) sowie Polyphthalamid (PPA). Geeignet sind auch die Hochleistungsthermoplaste Polyetherketon (PEK), Poly(etheretherketon) (PEEK), Poly(etherketonketon) (PEKK), Poly(etheretheretherketon) (PEEEK), Poly(etheretherketonketon) (PEEKK), Poly(etherketon-etherketonketon) (PEKEKK) sowie Polyaryletherketone (PAEK). Außerdem können auch faserverstärkte Kunststoffe verwendet werden.

In einer anderen vorteilhaften Ausgestaltung eines Aspekts der Erfindung ist der Quotient des Transmissionsgrads des Bereichs der Wandung wenigstens in dem zweiten Temperaturbereich für Strahlung der zweiten Wellenlänge oder des zweiten Wellenlängenbereichs und des Transmissionsgrads der Wandung in wenigstens dem ersten Temperaturbereich für Strahlung wenigstens der ersten Wellenlänge oder des ersten Wellenlängenbereichs größer als 5, bevorzugt größer als 10, besonders bevorzugt größer als 50.

In einer anderen vorteilhaften Ausgestaltung eines Aspekts der Erfindung weist die Wandung einen Durchlassbereich auf, der wenigstens in dem zweiten Temperaturbereich für Strahlung der zweiten Wellenlänge oder des zweiten Wellenlängenbereichs durchlässig ist, wobei die Wandung ferner auf einer dem Durchlassbereich gegenüberliegenden Seite einen Reflektionsbereich aufweist, der wenigstens in dem zweiten Temperaturbereich dazu ausgestaltet ist, durch den Hohlraum durchtretende Strahlung der zweiten Wellenlänge oder des zweiten Wellenlängenbereichs mit einem Reflektionsgrad von wenigstens 50% in den Hohlraum zu reflektieren oder zu zurückzustreuen.

Wird durch den Reflektionsbereich die Strahlung, die durch das Dentalmaterial hindurchgetreten ist, also noch nicht absorbiert wurde, nochmals infolge der Reflektion durch das Dentalmaterial geschickt, erhöht sich damit insgesamt die Wärmeausbeute und der Wirkungsgrad der Heizung per Infrarot-Strahlung wird verbessert.

Der Reflektionsbereich kann, vorzugsweise im Kontakt mit dem Dentalmaterial, also auf der Innenseite der Wandung (als Beschichtung oder eingebettet), ein anderes thermochromes Material aufweisen, dessen Schalttemperatur mit der Temperatur abgestimmt ist, auf die das Dentalmaterial erwärmt werden soll (z.B. an der Untergrenze oder der Obergrenze des zweiten Temperaturbereichs oder auch darin liegt), wobei das andere thermochrome Material einen Reflektionsgrad von wenigstens 50% für die Strahlung der zweiten Wellenlänge oder des zweiten Wellenlängenbereichs aufweist. Diese andere thermochrome Material erfüllt damit einerseits den Zweck des Reflektionsbereichs und erlaubt andererseits eine visuelle Wahrnehmung der erreichten Temperatur, die durch den Farbumschlag angezeigt wird. Insbesondere wenn das andere thermochrome Material die Infrarot-Strahlung kaum oder gar nicht selbst absorbiert, ergibt sich dessen Temperatur aus der Temperatur des im Hohlraum daran anschließenden Dentalmaterials.

In einer anderen vorteilhaften Ausgestaltung eines Aspekts der Erfindung ist der erste Temperaturbereich ein Bereich von 15 bis 25°C ist und der zweite Temperaturbereich ein Bereich von 25 bis 75°C.

Unabhängig von den hier als vorteilhaft angegebenen Temperaturbereichen ist es nicht notwendig, dass der erste Temperaturbereich direkt an den zweiten Temperaturbereich anschließt, auch wenn dies in bevorzugten Ausführungen der Erfindung vorgesehen ist.

In einer anderen vorteilhaften Ausgestaltung eines Aspekts der Erfindung umfasst die Vorrichtung im Hohlraum ein strahlungshärtbares Dentalmaterial, wobei das Dentalmaterial mit der Strahlung der ersten Wellenlänge oder des ersten Wellenlängenbereich zu härten ist, wobei das Dentalmaterial in dem ersten Temperaturbereich, insbesondere bei einer Temperatur von 20°C, eine Viskosität von größer als 400 Pa·s besitzt und wobei das Dentalmaterial in dem zweiten Temperaturbereich, insbesondere bei einer Temperatur von 60°C, eine Viskosität von niedriger als 150 Pa·s besitzt.

Vorzugsweise besitzt das Dentalmaterial im ersten Temperaturbereich, insbesondere bei einer Temperatur von 20°C, eine Viskosität von größer als 800 Pa·s, besonders bevorzugt von größer als 1.200 Pa·s.

Vorzugsweise besitzt das Dentalmaterial im zweiten Temperaturbereich, insbesondere bei einer Temperatur von 60°C, eine Viskosität von niedriger als 120 Pa·s, besonders bevorzugt von niedriger als 90 Pa·s.

Es ist hierbei zudem bevorzugt, wenn der Quotient aus der Viskosität des Dentalmaterials im zweiten Temperaturbereich (etwa bei 60°C) und der Viskosität des Dentalmaterials im ersten Temperaturbereich (etwa bei 20°C) kleiner als 0,125, besonders bevorzugt kleiner als 0,1 ist.

In einer anderen vorteilhaften Ausgestaltung eines Aspekts der Erfindung umfasst die Vorrichtung im Hohlraum ein strahlungshärtbares Dentalmaterial, welches eine einkomponentige Kompositzusammensetzung umfassend (A) Monomere, (B) Füllstoffe und (C) Initiatoren ist.

Die dentale, lichthärtbare, einkomponentige Kompositzusammensetzung vorzugsweise zur Herstellung eines dentalen Füllungsmaterials, Unterfüllungsmaterials, Befestigungsmaterials oder Fissurenversieglers, kann hierbei beispielsweise umfassen:
(A) Monomere in einer Menge von 6 bis 35 Gew.-%, bezogen auf die Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt 75 bis 89 Gew.-%, bezogen auf die Kompositzusammensetzung,
(C) Initiatoren in einer Menge von 0,001 bis 3 Gew.-%, bezogen auf die Menge der Kompositzusammensetzung,
(D) weitere Additive in einer Menge von 0,001 bis 5 Gew.-%, bezogen auf die Menge der Kompositzusammensetzung.

In einer besonderen Variante umfasst Bestandteil (A) der dentalen, lichthärtbaren, einkomponentigen Kompositzusammensetzung die Mischung von zumindest (A-i) einer ersten Monomerensubstanz und (A-ii) einer zweiten Monomerensubstanz, wobei die Viskosität der zweiten Monomerensubstanz (A-ii) bei 20 °C größer ist als 100 Pa·s, die Viskosität der ersten Monomerensubstanz (A-i) bei 20 °C größer ist als 100 mPa·s, die Viskosität der zweiten Monomerensubstanz (A-ii) bei 20 °C größer ist als die der ersten Monomerensubstanz (A-i) und das Massenverhältnis der ersten Monomerensubstanz (A-i) zur zweiten Monomerensubstanz (A-ii) im Bereich von 2:1 bis 1:10 liegt, wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist.

Die Untersuchungen, welche die Erfinder im Rahmen der vorliegenden Erfindung durchgeführt haben, zeigen, dass bei Einsatz der Bestandteile (A) bis (D) in den angegebenen Mengen (vorzugsweise in den als bevorzugt angegebenen Mengenbereichen) besonders effizient vorgesehenen Viskositätsmaßgaben eingestellt werden können.

Hierbei entspricht
(A1) den lichthärtbaren bi- oder tricyclischen Verbindungen Q(YₓZₑ)_{b},
(A2) dem Bis-GMA (2,2-Bis[4-(2-hydroxy-3-methacryloyloxypropoxy)phenyl]propan) und/oder den lichthärtbaren Derivaten des MDI (Diisocyanatodiphenylmethans) und/oder den lichthärtbare Derivate des TMXDI,
(A3) den lichthärtbaren Monomeren, die eine, zwei oder mehr ethylenische Gruppen aufweisende Substanzen sind, wie beispielsweise, ohne darauf beschränkt zu sein, die in der Dentalchemie üblicherweise eingesetzten (Meth)acrylatmonomere und die nicht (A1) und (A2) zuzuordnen sind,
(A4) den lichthärtbaren bi- oder tricyclischen Verbindungen Q(YₓZₑ)_{b}, dem UDMA (7,7,9-Trimethyl-3,14-dioxa-4,13-dioxo-5,12-diazahexadecan-1,16-dioxydi(meth)acrylat), dem BisEMA (alkoxyliertes Bisphenol-A-di(meth)acrylat mit n = 2 - 6), den hydroxylgruppenhaltigen Poly(meth)acrylaten, den alkoxylierten hydroxylgruppenhaltigen Poly(meth)acrylaten und den lichthärtbaren kettenförmigen und/oder ringförmigen und/oder käfigförmigen Polysiloxanen,
(A5) den lichthärtbaren Monomeren (A3) ohne (A4).

In einer bevorzugten Ausgestaltung bestehen die Monomere (A) aus
(A1) 10 bis 60 Gew.-%, vorzugsweise 20 bis 50 Gew.-%, besonders bevorzugt 25 bis 40 Gew.-%, lichthärtbare bi- oder tricyclische Verbindungen Q(YₓZₑ)_{b}, wobei gilt
   Q bedeutet ein gesättigtes oder olefinisch ungesättigtes bi- oder tricyclisches Strukturelement, jeder Index b ist eine natürliche Zahl, ausgewählt aus der Gruppe der natürlichen Zahlen 1, 2, und 3, jedes Z bedeutet eine lichthärtbare Gruppe, jeder Index e ist eine natürliche Zahl, ausgewählt aus der Gruppe der natürlichen Zahlen 1, 2 und 3, jedes Y bedeutet in der Struktur Q(YₓZₑ)_{b} bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und jeder Index x ist 0 oder 1,
(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-% weitere radikalisch polymerisierbare Monomere, die nicht (A1) oder (A2) zuzuordnen sind,
wobei die Gew.-%-Angaben von (A1), (A2) und (A3) auf die Gesamtmasse der Monomere (A) bezogen sind.

Die dentale, lichthärtbare, einkomponentige Kompositzusammensetzung kann ferner beispielsweise umfassen (A) Monomere, (B) Füllstoffe und, (C) Initiatoren, wobei die Monomere (A) bestehen aus
(A1) 10 bis 60 Gew.-%, vorzugsweise 20 bis 50 Gew.-%, besonders bevorzugt 25 bis 40 Gew.-%, lichthärtbare bi- oder tricyclische Verbindungen Q(YₓZₑ)_{b}, wobei gilt, Q bedeutet ein gesättigtes oder olefinisch ungesättigtes bi- oder tricyclisches Strukturelement,jeder Index b ist eine natürliche Zahl, ausgewählt aus der Gruppe der natürlichen Zahlen 1, 2, und 3, jedes Z bedeutet eine lichthärtbare Gruppe, jeder Index e ist eine natürliche Zahl, ausgewählt aus der Gruppe der natürlichen Zahlen 1, 2 und 3, jedes Y bedeutet in der Struktur Q(YₓZₑ)_{b} bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und jeder Index x ist 0 oder 1,
(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A1) oder (A2) zuzuordnen sind,
wobei die Gew.-%-Angaben von (A1), (A2) und (A3) auf die Gesamtmasse der Monomere (A) bezogen sind.

In einer bevorzugten Variante der beiden vorstehenden Ausgestaltungen weist das Dentalmaterial eine Absorberkomponente auf, die zumindest in dem zweiten Temperaturbereich einen Absorptionsgrad für Strahlung der zweiten Wellenlänge oder des zweiten Wellenlängenbereichs von mehr als 50% besitzt.

Ist das Dentalmaterial mit einer Absorberkomponente versehen, die in das Dentalmaterial eingearbeitet, darin eingebettet oder darin enthalten ist, wirkt dieses Absorbermaterial gewissermaßen wie eine lokale Heizung im Dentalmaterial. Selbst wenn ein bekanntes Dentalmaterial an sich schon eine gewisse Absorption von Strahlung der zweiten Wellenlänge oder des zweiten Wellenlängenbereichs besitzt, kann dies durch die Absorberkomponente verbessert werden, wobei die gesonderte Absorberkomponente zudem einen zusätzlichen Freiheitsgrad bei der Materialauswahl des eigentlichen Dentalmaterials erlaubt, als dass bei dieser Materialauswahl nicht oder zumindest weniger auf die Absorptionseigenschaften für die Strahlung der zweiten Wellenlänge oder des zweiten Wellenlängenbereichs geachtet werden muss.

Die Absorberkomponente sollte idealerweise im sichtbaren Bereich keine oder nur eine unwesentliche Absorption aufweisen, da sich ansonsten eine erwünschte Färbung des Dentalmaterials ergeben kann bzw. die Absorberkomponente nur in geringerem Umfang eingesetzt werden kann.

Ein Beispiel einer solchen Absorberkomponente umfasst ein wärmeabsorbierendes Glas mit einem Gehalt als Eisenoxid von 0,2% (bevorzugt im Bereich von 2% bis 4%) oder mehr (berechnet als FeO), wobei bei diesem Glas im Vergleich zu einem Silicatglas, das neben einwertigen, zweiwertigen und dreiwertigen glasbildenden Oxiden mindestens 25% Kieselsäure (SiO₂) enthält, der Kieselsäuregehalt ganz oder teilweise durch Phosphorsäure (P₂O₅) und/oder Borsäure (B₂O₃) ersetzt ist, wobei der Gehalt an P₂O₅ bzw. B₂O₃ bzw. P₂O₅ und B₂O₃ mindestens 25% beträgt. Wenn durch geeignete Maßnahmen (z.B. reduzierendes Schmelzen, reduzierende Zusätze; insbesondere Zufügen von Phosphor-Verbindungen niederer Oxidationsstufen bis herunter zu Phosphiden (einschließlich Phosphor) (z.B. Calciumhypophosphit (Ca[H₂PO₂]₂); Eisenphosphid (Fe₃P)) während eines Schmelzens und Abkühlens zur Erzielung weitgehender Reduktion) ein In-Lösung-Gehen des Eisenfarbstoffs als Eisenoxydul erreicht wird, ergibt sich somit ein Eisenoxydulphosphatglas, ein Eisenoxydulboratglas oder ein Eisenoxydulphosphatboratglas. Zur Verringerung einer Wasserlöslichkeit kann vorgesehen werden, dass das Glas 10% oder mehr an Tonerde (Al₂O₃) bzw. Aluminiumverbindungen entsprechend zu 10% oder mehr an Tonerde enthält. Vorzugsweise enthält das Glas weniger Kieselsäure als Phosphorsäure, Borsäure oder als die Kombination von Phosphorsäure und Borsäure.

Als Absorberkomponente kann beispielsweise auch ein wärmeabsorbierenden optisches Filtermaterial genutzt werden, wie es unter der Bezeichnung Schott KG5 erhältlich ist, wobei in geringerem Maße ebenfalls die Filtermaterialien KG4 bis KG1 eingesetzt werden können.

Auch wenn die obigen Beispiele eines Materials für die Absorberkomponente anorganischer Natur sind, ist die Erfindung nicht in dieser Hinsicht begrenzt und es ist möglich, dass auch eine organische Absorberkomponente Verwendung findet, wobei auch eine Kombination von anorganischen und organischen Materialien möglich ist.

In einer anderen vorteilhaften Ausgestaltung eines Aspekts der Erfindung weist die Vorrichtung in dem Hohlraum einen Absorptionsbereich auf, der ein Absorptionsmaterial aufweist oder daraus besteht, das zumindest in dem zweiten Temperaturbereich einen Absorptionsgrad für Strahlung der zweiten Wellenlänge oder des zweiten Wellenlängenbereichs von mehr als 50% besitzt.

Alternativ oder ergänzend zur Absorption durch das Dentalmaterial kann die Vorrichtung selbst mit einem "internen Heizelement" in Form des im Hohlraum angeordneten Absorptionsbereichs ausgestattet sein, wobei dieser Absorptionsbereich gegebenenfalls mit einer vergrößerten Oberfläche (z.B. in Form von Rippen) versehen sein kann, um so einen besonders guten Wärmeübergang auf das Dentalmaterial zu erlauben.

In einer anderen vorteilhaften Ausgestaltung eines Aspekts der Erfindung ist die Vorrichtung ausgewählt aus der Gruppe bestehend aus Spritzen, Applikationskanülen und Compulen.

In einer anderen vorteilhaften Ausgestaltung eines Aspekts der Erfindung weist die Vorrichtung eine Applikationsspitze zur Applikation des Dentalmaterials auf, wobei die Applikationsspitze eine Austrittsöffnung für das Dentalmaterial besitzt, wobei die Austrittsöffnung vorzugsweise eine Austrittsquerschnittsfläche im Bereich von 0,2 bis 3,0 mm², vorzugsweise von nicht mehr als 2,0 mm², besonders vorzugsweise von nicht mehr als 1,5 mm² besitzt.

Im ersten Temperaturbereich erlaubt die ausreichend hohen Viskosität eine tropfsichere Lagerung und Haltung, so dass beispielsweise eine Kontamination von Arbeitsflächen vermieden werden kann. Andererseits bringt die Erwärmung (z.B. auf 40 °C bis 80°C) eine gezielt verringerte Viskosität mit sich, die erlaubt, die Vorrichtung mit einer Applikationsspitze mit einer vergleichsweise kleinen Austrittsquerschnittsfläche auszugestalten, um eine punktgenaue Applikation bei der Behandlung zu gewährleisten.

In einer vorteilhaften Ausgestaltung eines Aspekts der Erfindung wird durch die Wandung der bereitgestellten Vorrichtung vor dem Bestrahlen mit der Strahlung der zweiten Wellenlänge oder des zweiten Wellenlängenbereichs und während des Bestrahlens mit der Strahlung der zweiten Wellenlänge oder des zweiten Wellenlängenbereichs Strahlung der ersten Wellenlänge oder des ersten Wellenlängenbereichs blockiert und/oder reflektiert und Strahlung der zweiten Wellenlänge oder des zweiten Wellenlängenbereichs zum Dentalmaterial durchgelassen.

In einer anderen vorteilhaften Ausgestaltung eines Aspekts der Erfindung ist als weiterer Schritt ein Ausbringen des Dentalmaterials aus der Vorrichtung vorgesehen.

In einer bevorzugten Variante der obigen Ausgestaltung ist als weiterer Schritt ein Bestrahlen des Dentalmaterials mit Strahlung der ersten Wellenlänge oder des ersten Wellenlängenbereichs vorgesehen, so dass das Dentalmaterial aushärtet.

In einer anderen vorteilhaften Ausgestaltung eines Aspekts der vorliegenden Offenbarung in Form eines dentalen therapeutischen Verfahrens ist als weiterer Schritt ein Ausbringen des Dentalmaterials aus der Vorrichtung vorgesehen, wobei das Dentalmaterial beim oder nach dem Ausbringen aus der Vorrichtung in die Mundhöhle eines Patienten eingebracht wird und vorzugsweise dort abkühlen gelassen wird und/oder danach so mit Strahlung der ersten Wellenlänge oder des ersten Wellenlängenbereichs bestrahlt wird, dass es aushärtet. Dieser Aspekt ist von den Ansprüchen nicht erfasst.

In einer anderen vorteilhaften Ausgestaltung eines Aspekts der vorliegenden Offenbarung in Form eines dentalen therapeutischen Verfahrens wird das Dentalmaterial mit einem zu behandelnden Zahn eines Patienten kontaktiert, vorzugsweise als Füllungsmaterial, Unterfüllungsmaterial, Befestigungsmaterial oder Fissurenversiegler. Dieser Aspekt ist von den Ansprüchen nicht erfasst.

Erfindungsgemäß wird nach einem weiteren Aspekt eine Verwendung eines Filtermaterials zur Herstellung einer erfindungsgemäßen Vorrichtung mit einem solchen Filtermaterial vorgeschlagen.

Erfindungsgemäß wird nach einem weiteren Aspekt eine Verwendung eines thermochromen Materials zur Herstellung einer erfindungsgemäßen Vorrichtung vorgeschlagen.

Offenbart wird auch eine Verwendung eines Absorbermaterials zur Herstellung einer erfindungsgemäßen Vorrichtung vorgeschlagen, bei der das Absorbermaterial als Absorperkomponente in das strahlungshärtbare Dentalmaterial eingebracht ist.

Erfindungsgemäß wird nach einem weiteren Aspekt eine dentale Anlage oder ein Kit mit einer erfindungsgemäßen Vorrichtung und einer Strahlungsquelle für die Strahlung der zweiten Wellenlänge oder des zweiten Wellenlängenbereichs vorgeschlagen, wobei die dentale Anlage oder das Kit ferner einer Strahlungsquelle für die Strahlung der ersten Wellenlänge oder des ersten Wellenlängenbereichs aufweisen kann.

Merkmale vorteilhafter Ausführungsformen der Erfindung sind insbesondere in den Unteransprüchen definiert, wobei weitere vorteilhafte Merkmale, Ausführungen und Ausgestaltungen für den Fachmann zudem aus den obigen Erläuterung und der folgenden Diskussion zu entnehmen sind.

Im Folgenden wird die vorliegende Erfindung anhand von in den Figuren dargestellten Ausführungsbeispielen weiter illustriert und erläutert. Hierbei zeigt
- Fig. 1: eine schematische Darstellung zur Illustration eines ersten Ausführungsbeispiels der erfindungsgemäßen Vorrichtung,
- Fig. 2: eine weitere schematische Darstellung zur Illustration eines ersten Ausführungsbeispiels der erfindungsgemäßen Vorrichtung und deren Verwendung,
- Fig. 3: eine weitere schematische Darstellung zur Illustration eines ersten Ausführungsbeispiels der erfindungsgemäßen Vorrichtung und deren Verwendung,
- Fig. 4: eine weitere schematische Darstellung zur Illustration eines ersten Ausführungsbeispiels der erfindungsgemäßen Vorrichtung und deren Verwendung,
- Fig. 5: eine weitere schematische Darstellung zur Illustration eines ersten Ausführungsbeispiels der erfindungsgemäßen Vorrichtung und deren Verwendung,
- Fig. 6: eine schematische Darstellung zur Illustration eines zweiten Ausführungsbeispiels der erfindungsgemäßen Vorrichtung,
- Fig. 7: ein schematisches Ablaufdiagramm eines ersten Ausführungsbeispiels des erfindungsgemäßen Verfahrens zum Behandeln eines Dentalmaterials und
- Fig. 8: ein schematisches Ablaufdiagramm eines zweiten Ausführungsbeispiels des erfindungsgemäßen Verfahrens zum Behandeln eines Dentalmaterials.

In den beiliegenden Zeichnungen sowie den Erläuterungen zu diesen Zeichnungen sind einander entsprechende bzw. in Beziehung stehende Elemente - soweit zweckdienlich - mit jeweils entsprechenden oder ähnlichen Bezugszeichen gekennzeichnet, auch wenn sie in unterschiedlichen Ausführungsbeispielen zu finden sind.

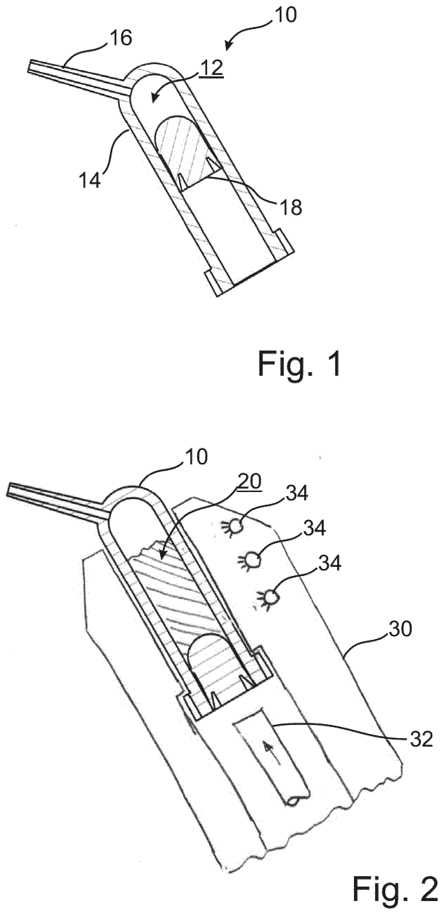

Fig. 1 zeigt eine schematische Darstellung zur Illustration eines ersten Ausführungsbeispiels der erfindungsgemäßen Vorrichtung.

Im ersten Ausführungsbeispiel ist die Vorrichtung 10 als Compule ausgeführt, die einen Hohlraum 12 aufweist, der zur Aufnahme von Dentalmaterial (in Fig. 1 nicht gezeigt) vorgesehen ist. Der Hohlraum 12 wird von einer Wandung 14 der Compule 10 umschlossen, die wiederum mit einer Applikationsspitze 16 versehen ist. Der zur Verfügung stehende Teil des Hohlraums 12 wird zudem von einem Kolben 18 begrenzt, der innerhalb der Wandung 14 der Compule 10 angeordnet ist, um entlang einer Längsachse der Compule 10 beweglich zu sein, so dass im Hohlraum 12 befindliches Dentalmaterial durch die Applikationsspitze 16 ausgebracht werden kann.

Da Compulen als solche, ihr Aufbau und ihre Verwendung bekannt sind, kann hier auf eine weitere Erläuterung verzichtet werden.

Es sei allerdings darauf hingewiesen, dass die Beschreibung der Erfindung anhand von Ausführungsbeispielen in Form einer Compule nicht als Beschränkung zu verstehen ist, da die Erfindung allgemein eine zur Aufnahme und Applikation von Dentalmaterial geeignete Vorrichtung vorsieht, die nicht nur durch eine Compule realisiert werden kann, sondern beispielsweise auch durch eine Spritze oder eine Applikationskanüle.

Fig. 2 zeigt eine weitere schematische Darstellung zur Illustration eines ersten Ausführungsbeispiels der erfindungsgemäßen Vorrichtung und deren Verwendung.

Die in Fig. 1 gezeigte Compule 10 ist hier mit im Hohlraum 12 aufgenommenem Dentalmaterial 20 gezeigt, wobei sich der Kolben 18 zudem in einer Ausgangsposition befindet, d.h. maximal weit von der Applikationsspitze 16 entfernt ist, so dass der für das Dentalmaterial 20 zur Verfügung stehenden Teil des Hohlraums 12 maximal wird. In der Darstellung von Fig. 2 ist ein an die Applikationsspitze 16 anschießender Bereich des Hohlraums 12 nicht mit Dentalmaterial 20 gefüllt, wobei allerdings auch eine vollständige Füllung des Hohlraums vorgesehen sein kann. Im ersten Temperaturbereich (z.B. bei 20°C) besitzt das Dentalmaterial vorzugweise eine so hohe Viskosität, dass die Applikationsspitze 16 einen für ein Austreten zu großen Widerstand bietet.

Die Compule 10 ist in einer Applikationsspitze einer Compulenzange 30 aufgenommen, wobei die Compulenzange 30 in an sich bekannter Weise einen Stempel 32 zum Vortreiben des Kolbens 18 bei ansonsten von der Compulenzange 30 gehaltener Compule 10 aufweist. Die Compulenzange 30 ist zudem im Bereich der gehaltenen Compule 10 mit mehreren Strahlungsquellen in Form von Infrarot-LEDs 34 ausgestattet.

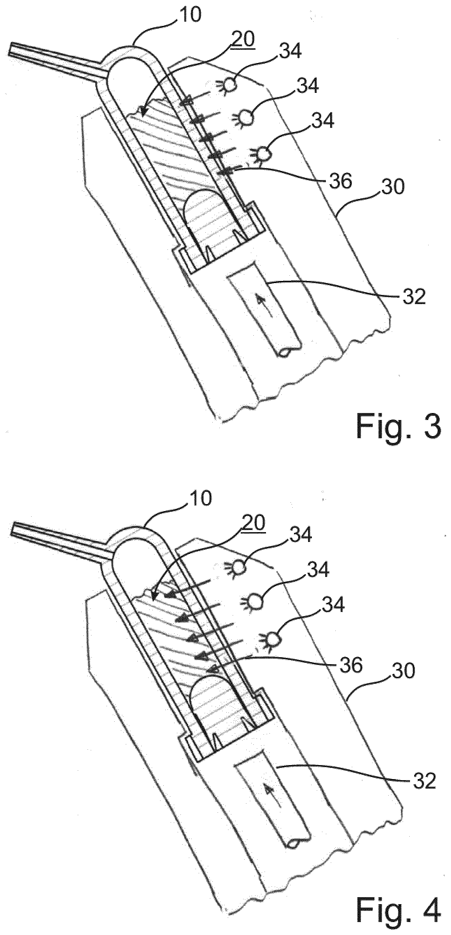

Fig. 3 zeigt eine weitere schematische Darstellung zur Illustration eines ersten Ausführungsbeispiels der erfindungsgemäßen Vorrichtung und deren Verwendung.

Die Darstellung von Fig. 3 entspricht der von Fig. 2, wobei in Fig. 3 allerdings die Situation illustriert ist, dass durch die IR-LEDs 34 der Compulenzange 30 Strahlung 36 im Infrarot-Bereich (insbesondere im nahen Infrarot-Bereich) auf die Compule 10 eingestrahlt wird.

Im vorliegenden Ausführungsbeispiel umfasst die Wandung 14 der Compule 10 ein thermochromes Material, das z.B. bei 20°C für die eingestrahlte Infrarot-Strahlung undurchlässig ist. Zudem ist die Wandung 14 für Strahlung für die Wellenlänge oder Wellenlängen undurchlässig, die zum Lichthärten des Dentalmaterials 20 genutzt werden kann. Dies ist insofern von Bedeutung, dass damit ein mit der eigentlichen Compule 10 integrierter Schutz für den darin enthaltene Dentalmaterial 20 gegeben ist, so dass die Compule 10 ohne die Sorge gehandhabt werden kann, dass durch eine ungewollte Lichteinstrahlung ins Innere die Härtung des Dentalmaterials 20 ausgelöst wird.

Die von den IR-LEDs 34 in die Wandung 14 eingestrahlte Infrarot-Strahlung erwärmt die Wandung 14, solange die Strahlung dort zumindest zum Teil absorbiert wird. Es dürfte hierbei klar sein, dass der Energieeintrag durch die IR-LEDs 34 ausreichend groß sein muss, um eventuelle Verluste durch Wärmeleitung, Abstrahlung, usw. zu übersteigen.

Eine bevorzugte Kombination der in der vorliegenden Anmeldung diskutierten Aspekte liegt insbesondere bei einer Vorrichtung (z.B. in Form einer Compule) aus Polyamid mit den oben erwähnten Perylenderivate (5) und (6) als Filtermaterial und einer Strahlung im nahen Infrarot-Bereich zur Erwärmung (insbesondere mit einer Wellenlänge von 800 bis 1000 nm) vor, wobei dann bevorzugt die Monomere (A) des Dentalmaterials aus den mit (A1) bis (A3) genannten Materials gegeben sind.

Fig. 4 zeigt eine weitere schematische Darstellung zur Illustration eines ersten Ausführungsbeispiels der erfindungsgemäßen Vorrichtung und deren Verwendung.

Die Darstellung von Fig. 4 entspricht, wie schon die von Fig. 3, der von Fig. 2, wobei in Fig. 4 allerdings die Situation illustriert ist, dass durch die IR-LEDs 34 der Compulenzange 30 bereits so viel Strahlung 36 im Infrarot-Bereich (insbesondere im nahen Infrarot-Bereich) auf die Compule 10 eingestrahlt wurde (wie es in Fig. 3 angedeutet ist), dass das thermochrome Material über seine Schalttemperatur hinaus erwärmt wurde und damit für die Infrarot-Strahlung durchlässig(er) wurde.

Wie es in Fig. 4 dargestellt ist, gelangt die Infrarot-Strahlung nun direkt in das Dentalmaterial 20, das somit erwärmt werden kann.

Es ist nicht notwendig, dass die komplette Wandung 14 eine Durchlässigkeit für die Infrarot-Strahlung besitzt oder annimmt, solange ein ausreichend großer Bereich der Wandung (der nicht notwendigerweise durchgehend sein muss) die nötige Durchlässigkeit zeigt.

Ebenso ist es nicht notwendigerweise der Fall, dass das gesamte in der Wandung 14 oder dem Bereich der Wandung 14 vorliegende thermochrome Material über die Schalttemperatur hinaus erwärmt wird, solange ein gewünschter ausreichender Wärmeeintrag in das Dentalmaterial 20 sichergestellt ist.

Es kann zudem vorgesehen sein (nicht gezeigt), dass die Compule 10 auf oder in der Wandung 14 auf der den IR-LEDs 34 gegenüberliegenden Seite einen Reflektionsbereich für die genutzte Strahlung 36 aufweist, die damit wiederum zurück in das Dentalmaterial 20 reflektiert wird.

Bei Ermöglichung einer entsprechenden Sichtbarkeit (z.B. durch eine Öffnung oder Aussparung in der Compulenzange 30) kann die Wandung 14 zudem vorteilhafterweise mit einem weiteren thermochromen Material versehen sein, dessen Schalttemperatur auf die gewünschte Temperatur des Dentalmaterials 20 abgestimmt ist, so dass mit dem Farbumschlag das Erreichen der gewünschten Temperatur erkennbar gemacht wird. Weist das weitere thermochrome Material Reflektionseigenschaften für die genutzte Infrarot-Strahlung auf, kann es auch für den oben erwähnten Reflektionsbereich genutzt werden.

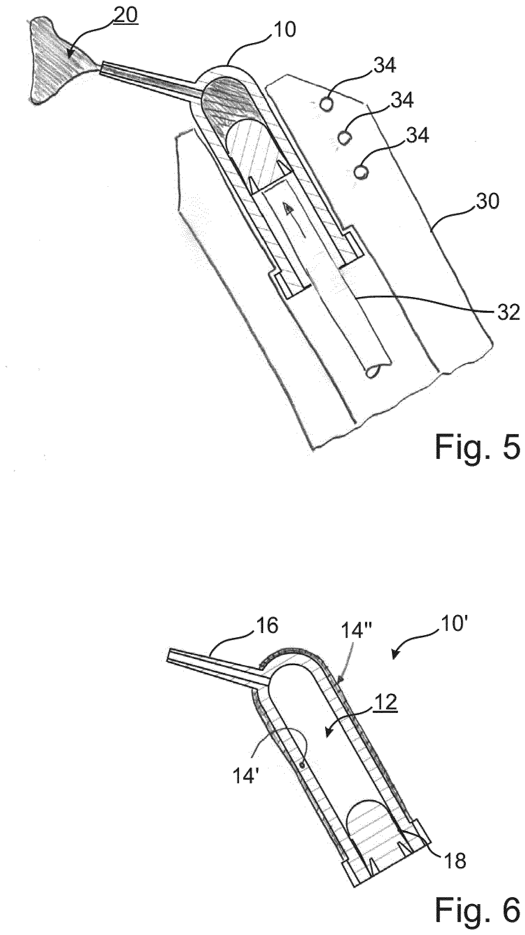

Fig. 5 zeigt eine weitere schematische Darstellung zur Illustration eines ersten Ausführungsbeispiels der erfindungsgemäßen Vorrichtung und deren Verwendung.

Fig. 5 schließt an die Darstellung von Fig. 4 an. Nachdem unter Nutzung der IR-LEDs 34 durch die Wandung 14 der Compule 10 das darin enthaltene Dentalmaterial 20 ausreichend erwärmt wurde, d.h. eine gewünschte verringerte Viskosität angenommen hat, wird durch ein Vortreiben des Stempel 32 über den Kolben 18 das Dentalmaterial 20 durch die Applikationsspitze 16 der Compule 10 ausgetrieben.

Fig. 6 zeigt eine schematische Darstellung zur Illustration eines zweiten Ausführungsbeispiels der erfindungsgemäßen Vorrichtung.

Auch im zweiten Ausführungsbeispiel ist die Vorrichtung 10' als Compule ausgeführt, die einen Hohlraum 12 aufweist, der zur Aufnahme von Dentalmaterial (in Fig. 6 nicht gezeigt) vorgesehen ist. Der Hohlraum 12 wird von einer Wandung der Compule 10' umschlossen, die wiederum mit einer Applikationsspitze 16 versehen ist. Der zur Verfügung stehende Teil des Hohlraums 12 wird zudem von einem Kolben 18 begrenzt, der innerhalb der Wandung 14' der Compule 10' angeordnet ist, um entlang einer Längsachse der Compule 10' beweglich zu sein, so dass im Hohlraum 12 befindliches Dentalmaterial durch die Applikationsspitze 16 ausgebracht werden kann.

Im Gegensatz zum ersten Ausführungsbeispiel, das in den Fig. 1 bis 5 illustriert ist und bei dem das thermochrome Material in dem Material einer im Wesentlichen homogen (oder zumindest einschichtigen) Wandung 14 der Compule 10 selbst enthalten ist, ist beim zweiten Ausführungsbeispiel die Wandung schichtweise ausgebildet, wobei im in Fig. 6 gezeigten Ausführungsbeispiel eine herkömmliche Compule mit einer Wandungsschicht 14' mit einer zusätzlichen Außenschicht 14" versehen ist, die das thermochrome Material umfasst.

Die Außenschicht 14" bildet zusammen mit der Wandungsschicht 14' die Wandung der Compule 10' und kann beispielsweise in Form einer die Wandungsschicht 14' überziehenden Folie, eines Lacks oder Etiketts oder durch einen Schrumpfschlauch gebildet werden.

Ein Anbringen an der Innenseite der Wandungsschicht ist ebenfalls denkbar, wobei auch eine Schichtung von drei oder mehr Schichten möglich ist.

In den beiden oben diskutierten Ausführungsbeispielen ist jeweils in oder an der Wandung oder Wandungsschicht thermochromes Material vorgesehen. Mit Ausnahme von Fig. 3 gelten die obigen Ausführungen allerdings auch in entsprechend angepasster Weise für den Fall, dass ein Filtermaterial vorgesehen ist. Ebenso ist es möglich, eine Kombination von Filtermaterial und thermochromen Material vorzusehen.

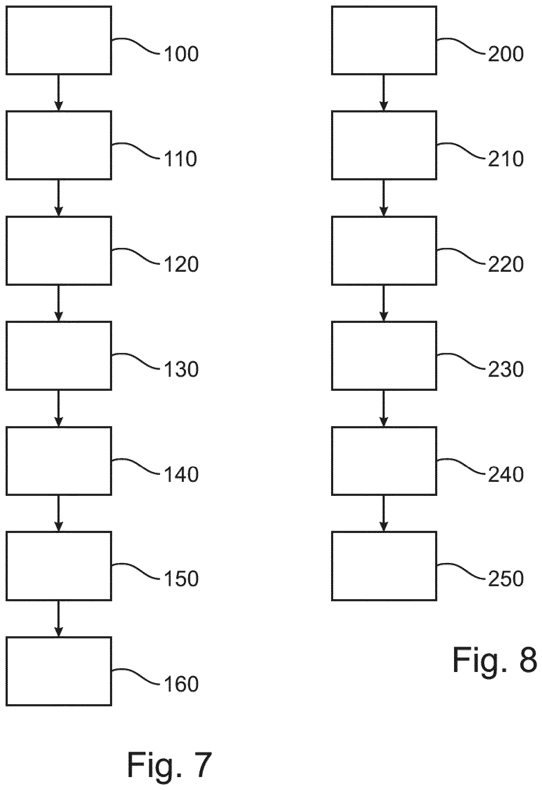

Fig. 7 zeigt ein schematisches Ablaufdiagramm eines ersten Ausführungsbeispiels des erfindungsgemäßen Verfahrens zum Behandeln eines Dentalmaterials.

In Schritt 100 wird zunächst eine erfindungsgemäße Vorrichtung mit darin aufgenommenen strahlungshärtbaren Dentalmaterial bereitgestellt, wobei das Dentalmaterial und die Vorrichtung jeweils eine Temperatur von 20°C aufweisen.

Die bereitgestellte Vorrichtung schützt durch die Undurchlässigkeit für etwa blaues Licht, das für die Härtung des Dentalmaterials vorgesehen ist, das Dentalmaterial vor einer ungewollten Initiierung des Härtungsprozesses.

In Schritt 110 wird die Vorrichtung mit Infrarot-Strahlung im Bereich von 750 nm bis 3.000 nm bestrahlt, wobei die Infrarot-Strahlung zunächst von der Wandung der Vorrichtung bzw. dem darin enthaltenden thermochromen Material aufgenommen wird, das sich damit erwärmt.

In Schritt 120 wird die Schaltemperatur des thermochromen Materials erreicht, was dazu führt, dass das thermochrome Material für die Infrarot-Strahlung durchlässig wird, so dass in Schritt 130 das Dentalmaterial in der Vorrichtung von der Infrarot-Strahlung erreicht und damit erwärmt wird.

Mit der Erwärmung des Dentalmaterials verringert sind in Schritt 140 die Viskosität des Dentalmaterials bis zu einer gewünschten Viskosität oder weniger, was z.B. bei einer Temperatur von 50°C erreicht sein kann.

In Schritt 150 wird das Dentalmaterial, das die gewünschte Viskosität oder sogar eine noch geringere Viskosität aufweist, aus der Vorrichtung ausgebracht.

In Schritt 160 wird das ausgebrachte Dentalmaterial mit blauem Licht bestrahlt, um die Härtungsprozess anzustoßen und durchzuführen.

Fig. 8 zeigt ein schematisches Ablaufdiagramm eines zweiten Ausführungsbeispiels des erfindungsgemäßen Verfahrens zum Behandeln eines Dentalmaterials.

In Schritt 200 wird zunächst eine erfindungsgemäße Vorrichtung mit darin aufgenommenen strahlungshärtbaren Dentalmaterial bereitgestellt, wobei das Dentalmaterial und die Vorrichtung jeweils eine Temperatur von 20°C aufweisen.

Die bereitgestellte Vorrichtung schützt durch die Undurchlässigkeit für etwa blaues Licht, das für die Härtung des Dentalmaterials vorgesehen ist, das Dentalmaterial vor einer ungewollten Initiierung des Härtungsprozesses.

In Schritt 210 wird die Vorrichtung mit Infrarot-Strahlung im Bereich von 750 nm bis 3.000 nm bestrahlt.

Schon beim Bereitstellen der Vorrichtung in Schritt 200 und auch während der Bestrahlung mit Infrarot-Strahlung blockiert und/oder reflektiert die Wandung mit ihrem Filtermaterial Strahlung der ersten Wellenlänge oder des ersten Wellenlängenbereichs (z.B. blaues Licht), lässt aber - anders als im Fall von Fig. 7 - die Infrarot-Strahlung zum Dentalmaterial durch.

Da die Wandung für die Infrarot-Strahlung durchlässig wird, wird in Schritt 220 das Dentalmaterial in der Vorrichtung von der Infrarot-Strahlung erreicht und damit erwärmt.

Mit der Erwärmung des Dentalmaterials verringert sind in Schritt 230 die Viskosität des Dentalmaterials bis zu einer gewünschten Viskosität oder weniger, was z.B. bei einer Temperatur von 50°C erreicht sein kann.

In Schritt 240 wird das Dentalmaterial, das die gewünschte Viskosität oder sogar eine noch geringere Viskosität aufweist, aus der Vorrichtung ausgebracht.

In Schritt 250 wird das ausgebrachte Dentalmaterial mit blauem Licht bestrahlt, um die Härtungsprozess anzustoßen und durchzuführen.

Die in den Fig. 7 und 8 illustrierten Schritte erfolgen jeweils außerhalb des menschlichen oder tierischen Körpers und haben als solche zunächst auch keine therapeutische Wirkung oder überhaupt eine Interaktion mit einem menschlichen oder tierischen Körper. Im Rahmen der vorliegenden Erfindung kann allerdings auch ein therapeutischer Einsatz vorgesehen sein, bei dem z.B. die Härtung des Dentalmaterials im oder am Zahn in der Mundhöhle erfolgt.

Auch wenn in den Figuren verschiedene Aspekte oder Merkmale der Erfindung jeweils in Kombination gezeigt sind, ist für den Fachmann - soweit nicht anders angegeben - ersichtlich, dass die dargestellten und diskutieren Kombinationen nicht die einzig möglichen sind. Insbesondere können einander entsprechende Einheiten oder Merkmalskomplexe aus unterschiedlichen Ausführungsbeispielen miteinander ausgetauscht werden.

### Bezugszeichenliste

- 10, 10': Compule
- 12: Hohlraum
- 14: Wandung
- 14': Wandungsschicht
- 14": Außenschicht
- 16: Applikationsspitze
- 18: Kolben
- 20: Dentalmaterial
- 30: Compulenzange
- 32: Stempel
- 34: IR-LED

## Patentansprüche

1. Vorrichtung (10, 10') zur Aufnahme und Applikation von Dentalmaterial (20), mit einem Hohlraum (12) zur Aufnahme des Dentalmaterials (20) und
einer den Hohlraum (12) umgebenden Wandung (14),
wobei die Wandung (14) in wenigstens einem ersten Temperaturbereich für Strahlung wenigstens einer ersten Wellenlänge oder eines ersten Wellenlängenbereichs im Bereich von 100 nm bis 500 nm undurchlässig ist, und
wobei die Wandung (14) wenigstens einen Bereich mit einem Trägermaterial, bevorzugt einem Polyamid-Material und/oder einem Polybutylenterephthalat-Material, und einem Filtermaterial, das außen am Trägermaterial, innen am Trägermaterial und/oder zwischen wenigstens zwei Schichten des Trägermaterials vorgesehen ist und/oder in das Trägermaterial eingebettet ist, aufweist,
**gekennzeichnet dadurch, dass** das Filtermaterial ausgewählt ist aus der Gruppe bestehend aus:
- den Perylenderivaten 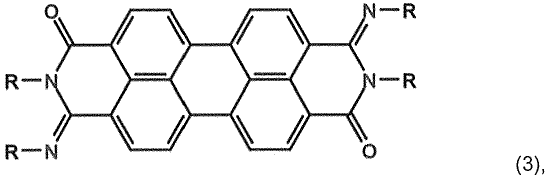 insbesondere 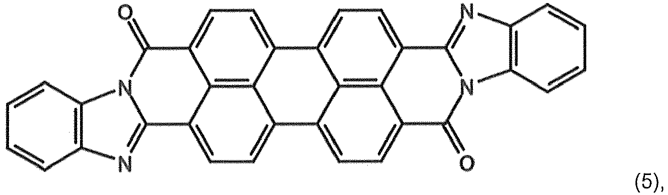 und 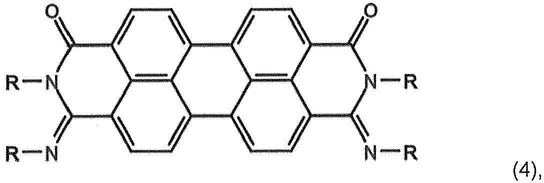 insbesondere 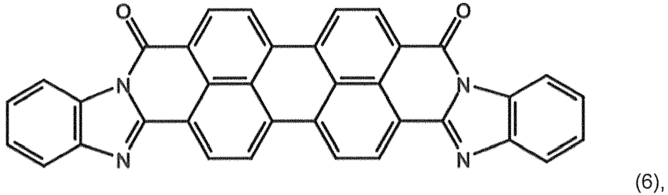 sowie Kombinationen davon.

2. Vorrichtung (10, 10') nach Anspruch 1,
wobei der Bereich der Wandung (14) ein Filtermaterial aufweist oder daraus besteht,
wobei das Filtermaterial zumindest im ersten Temperaturbereich, bevorzugt zumindest im ersten Temperaturbereich und in einem zweiten Temperaturbereich, für Strahlung der ersten Wellenlänge oder des ersten Wellenlängenbereichs einen Transmissionsgrad von weniger als 10%, bevorzugt von weniger als 5%, aufweist,
wobei das Filtermaterial zumindest im zweiten Temperaturbereich für Strahlung einer zweiten Wellenlänge oder eines zweiten Wellenlängenbereichs im Bereich von 600 nm bis 50.000 nm einen Transmissionsgrad von mehr als 50%, bevorzugt von mehr als 65%, besonders bevorzugt von mehr als 80% aufweist.

3. Vorrichtung (10, 10') nach Anspruch 2,
wobei das Filtermaterial ausgewählt ist aus der Gruppe bestehend aus Substanzen mit einem Perylen-Gerüst, aromatische Diazoverbindungen und Kombinationen davon.

4. Vorrichtung (10, 10') nach einem der vorangehenden Ansprüche,
wobei der Bereich der Wandung (14) thermochromes Material aufweist oder daraus besteht, das eine Schalttemperatur im Bereich von 25 bis 50°C besitzt,
wobei das thermochrome Material bei einer Temperatur unter der Schalttemperatur für Strahlung wenigstens einer zweiten Wellenlänge oder eines zweiten Wellenlängenbereichs im Bereich von 600 nm bis 50.000 nm einen niedrigeren Transmissionsgrad besitzt als bei einer Temperatur über der Schalttemperatur.

5. Vorrichtung (10, 10') nach Anspruch 4,
wobei das thermochrome Material ausgewählt ist aus der Gruppe bestehend aus anorganischen Pigmenten umfassend Metallsalze oder Metalloxide, bei denen durch einen Phasenübergang, eine Änderung der Ligandengeometrie, eine Änderung der Koordinationszahl und/oder eine Änderung des Kristallfeldes ein Farbübergang stattfindet, organischen Pigmenten umfassend thermochrome Flüssigkristalle, konjugierte Polymere und Leukofarbstoffe und Kombinationen davon.

6. Vorrichtung (10, 10') nach einem der vorangehenden Ansprüche,
wobei die Wandung (14) wenigstens ein Trägermaterial, bevorzugt ein Polyamid-Material und/oder ein Polybutylenterephthalat-Material, aufweist, wobei das Filtermaterial und/oder das thermochrome Material (14") außen am Trägermaterial (14'), innen am Trägermaterial und/oder zwischen wenigstens zwei Schichten des Trägermaterials vorgesehen ist und/oder in das Trägermaterial eingebettet ist, und/oder
wobei der Quotient des Transmissionsgrads des Bereichs der Wandung (14) wenigstens in einem zweiten Temperaturbereich für Strahlung einer zweiten Wellenlänge oder eines zweiten Wellenlängenbereichs im Bereich von 600 nm bis 50.000 nm und des Transmissionsgrads der Wandung (14) in wenigstens dem ersten Temperaturbereich für Strahlung wenigstens der ersten Wellenlänge oder des ersten Wellenlängenbereichs größer als 5, bevorzugt größer als 10, besonders bevorzugt größer als 50 ist, und/oder
wobei die Wandung (14) einen Durchlassbereich aufweist, der wenigstens in einem zweiten Temperaturbereich für Strahlung der zweiten Wellenlänge oder des zweiten Wellenlängenbereichs durchlässig ist, wobei die Wandung (14) ferner auf einer dem Durchlassbereich gegenüberliegenden Seite einen Reflektionsbereich aufweist, der wenigstens in dem zweiten Temperaturbereich dazu ausgestaltet ist, durch den Hohlraum (12) durchtretende Strahlung der zweiten Wellenlänge oder des zweiten Wellenlängenbereichs mit einem Reflektionsgrad von wenigstens 50% in den Hohlraum (12) zu reflektieren oder zu zurückzustreuen, und/oder
wobei der erste Temperaturbereich ein Bereich von 15 bis 25°C ist und der zweite Temperaturbereich ein Bereich von 25 bis 75°C ist.

7. Vorrichtung (10, 10') nach einem der vorstehenden Ansprüche, im Hohlraum (12) umfassend ein strahlungshärtbares Dentalmaterial (20),
wobei das Dentalmaterial (20) mit der Strahlung der ersten Wellenlänge oder des ersten Wellenlängenbereich zu härten ist, wobei das Dentalmaterial (20) in dem ersten Temperaturbereich, insbesondere bei einer Temperatur von 20°C, eine Viskosität von größer als 400 Pa·s besitzt und wobei das Dentalmaterial (20) in einem zweiten Temperaturbereich, insbesondere bei einer Temperatur von 60°C, eine Viskosität von niedriger als 150 Pa·s besitzt und/oder
wobei das strahlungshärtbare Dentalmaterial (20) eine einkomponentige Kompositzusammensetzung ist umfassend
(A) Monomere,
(B) Füllstoffe und
(C) Initiatoren.

8. Vorrichtung (10, 10') nach Anspruch 7,
wobei das Dentalmaterial (20) eine Absorberkomponente aufweist, die zumindest in dem zweiten Temperaturbereich einen Absorptionsgrad für Strahlung einer zweiten Wellenlänge oder eines zweiten Wellenlängenbereichs im Bereich von 600 nm bis 50.000 nm von mehr als 50% besitzt.

9. Vorrichtung (10, 10') nach einem der vorangehenden Ansprüche,
wobei die Vorrichtung (10, 10') in dem Hohlraum (12) einen Absorptionsbereich aufweist, der ein Absorptionsmaterial aufweist oder daraus besteht, das zumindest in einem zweiten Temperaturbereich einen Absorptionsgrad für Strahlung einer zweiten Wellenlänge oder eines zweiten Wellenlängenbereichs im Bereich von 600 nm bis 50.000 nm von mehr als 50% besitzt, und/oder
die Vorrichtung (10, 10') ausgewählt ist aus der Gruppe bestehend aus Spritzen, Applikationskanülen und Compulen und/oder
die Vorrichtung (10, 10') eine Applikationsspitze (16) zur Applikation des Dentalmaterials (20) aufweist, wobei die Applikationsspitze (16) eine Austrittsöffnung für das Dentalmaterial (20) besitzt, wobei die Austrittsöffnung vorzugsweise eine Austrittsquerschnittsfläche im Bereich von 0,2 bis 3,0 mm², vorzugsweise von nicht mehr als 2,0 mm², besonders vorzugsweise von nicht mehr als 1,5 mm² besitzt.

10. Verfahren zum Behandeln eines Dentalmaterials (20) außerhalb des menschlichen oder tierischen Körpers, mit folgenden Schritten:
- Bereitstellen (100, 200) einer Vorrichtung (10, 10') nach einem der Ansprüche 1 bis 9 umfassend strahlungshärtbares Dentalmaterial (20) in dem ersten Temperaturbereich,
- Bestrahlen (110, 210) der bereitgestellten Vorrichtung (10, 10') mit Strahlung einer zweiten Wellenlänge oder eines zweiten Wellenlängenbereichs, vorzugsweise mit IR-Licht einer Wellenlänge im Bereich von 600 nm bis 50.000 nm, zum Erwärmen (130, 220) des Dentalmaterials (20),
- Verringern (140, 230) der Viskosität des Dentalmaterials (20) durch die Erwärmung (130, 220) bis zu einer gewünschten Viskosität oder weniger.

11. Verfahren nach Anspruch 10, wobei durch die Wandung (14) der bereitgestellten Vorrichtung (10, 10') vordem Bestrahlen (110, 210) mit der Strahlung der zweiten Wellenlänge oder des zweiten Wellenlängenbereichs und während des Bestrahlens (110, 210) mit der Strahlung der zweiten Wellenlänge oder des zweiten Wellenlängenbereichs
- Strahlung der ersten Wellenlänge oder des ersten Wellenlängenbereichs blockiert und/oder reflektiert wird und
- Strahlung der zweiten Wellenlänge oder des zweiten Wellenlängenbereichs zum Dentalmaterial (20) durchgelassen wird.

12. Verfahren nach Anspruch 10 oder 11, mit folgendem weiteren Schritt:
- Ausbringen (150, 240) des Dentalmaterials (20) aus der Vorrichtung (10, 10').

13. Verfahren nach Anspruch 12, mit folgendem weiteren Schritt:
- Bestrahlen (160, 250) des Dentalmaterials (20) mit Strahlung der ersten Wellenlänge oder des ersten Wellenlängenbereichs, so dass das Dentalmaterial (20) aushärtet.

14. Verwendung eines Filtermaterials zur Herstellung einer Vorrichtung (10, 10') nach Anspruch 2 oder 3.

15. Dentale Anlage oder Kit, umfassend
- eine Vorrichtung (10, 10') nach einem der Ansprüche 1 bis 9 und
- eine Strahlungsquelle (34) für die Strahlung einer zweiten Wellenlänge oder eines zweiten Wellenlängenbereichs im Bereich von 600 nm bis 50.000 nm.

## Claims

1. Device (10, 10') for storage and application (20), comprising a cavity (12) for storage of the dental material (20) and
a wall (14) surrounding the cavity (12),
wherein the wall (14), in at least a first temperature range, is impermeable to radiation of at least a first wavelength or a first wavelength range in the range of 100 nm to 500 nm, and
wherein the wall (14) has at least one area with a carrier material, preferably a polyamide material and/or a polybutylene terephthalate material, and a filter material which is provided externally on the carrier material, internally on the carrier material and/or between at least two layers of the carrier material and/or is embedded in the carrier material,
**characterized in that** the filter material is selected from the group composed of:
- perylene derivatives 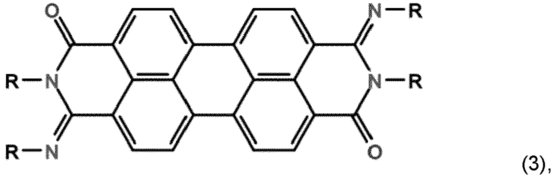 in particular 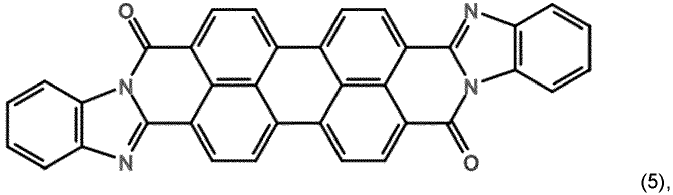 and 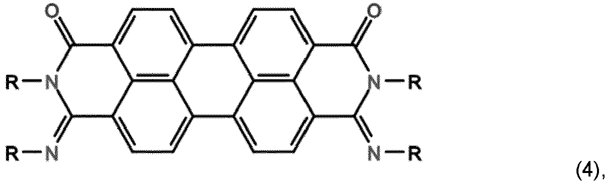 in particular 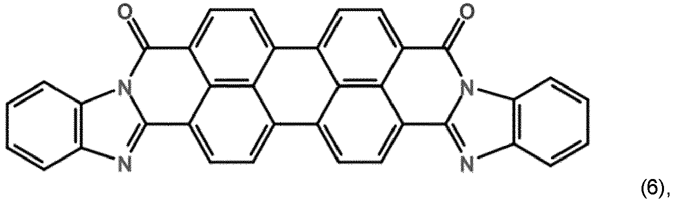 and combinations thereof.

2. Device (10, 10') according to Claim 1,
wherein the area of the wall (14) comprises or is composed of a filter material, wherein the filter material, at least in the first temperature range, preferably at least in the first temperature range and in a second temperature range, has a transmittance with respect to radiation of the first wavelength or the first wavelength range of less than 10%, and preferably less than 5%,
wherein the filter material, at least in the second temperature range, has a transmittance with respect to radiation of a second wavelength or a second wavelength range in the range of 600 nm to 50,000 nm of more than 50%, preferably more than 65%, and particularly preferably more than 80%.

3. Device (10, 10') according to Claim 2,
wherein the filter material is selected from the group composed of substances with a perylene skeleton, aromatic diazo compounds and combinations thereof.

4. Device (10, 10') according to one of the preceding claims,
wherein the area of the wall (14) comprises or is composed of thermochromic material having a switching temperature in the range of 25 to 50°C,
wherein the thermochromic material, at a temperature below the switching temperature, has lower transmittance with respect to radiation at least of a second wavelength or a second wavelength range in the range of 600 nm to 50,000 nm than at a temperature above the switching temperature.

5. Device (10, 10') according to Claim 4,
wherein the thermochromic material is selected from the group composed of inorganic pigments comprising metal salts or metal oxides in which a colour transition takes place due to a phase transmission, a change in the ligand geometry, a change in the coordination number and/or a change in the crystal field, organic pigments comprising thermochromic liquid crystals, conjugated polymers and leuco dyes and combinations thereof.

6. Device (10, 10') according to one of the preceding claims,
wherein the wall (14) comprises at least one carrier material, preferably a polyamide material and/or a polybutylene terephthalate material, wherein the filter material and/or the thermochromic material (14") is provided externally on the carrier material (14'), internally on the carrier material and/or between at least two layers of the carrier material and/or is embedded in the carrier material, and/or
wherein the quotient of the transmittance of the area of the wall (14), at least in a second temperature range, with respect to radiation of a second wavelength or a second wavelength range in the range of 600 nm to 50,000 nm and the transmittance of the wall (14) in at least the first temperature range with respect to radiation at least of the first wavelength or the first wavelength range is greater than 5, preferably greater than 10, and particularly preferably greater than 50, and/or
wherein the wall (14) has a transmission area which, at least in a second temperature range, is permeable to radiation of the second wavelength or the second wavelength range, wherein the wall (14) further has a reflection area on the side opposite the transmission area that is configured, at least in the second temperature range, to reflect or backscatter radiation of the second wavelength or the second wavelength range passing through the cavity (12) with a reflectance of at least 50% into the cavity (12), and/or
wherein the first temperature range is a range of 15 to 25°C and wherein the second temperature range is a range of 25 to 75°C.

7. Device (10, 10') according to one of the preceding claims, comprising in the cavity (12) a radiation-curable dental material (20),
wherein the dental material (20) is to be cured with the radiation of the first wavelength or the first wavelength range, wherein the dental material (20) in the first temperature range, in particular at a temperature of 20°C, has a viscosity of greater than 400 Pa·s and wherein the dental material (20) in a second temperature range, in particular at a temperature of 60°C, has a viscosity of less than 150 Pa·s, and/or,
wherein the radiation-curable dental material (20) is a single-component composite composition comprising
(A) monomers,
(B) fillers and
(C) initiators.

8. Device (10, 10') according to Claim 7,
wherein the dental material (20) comprises an absorber component which, at least in a second temperature range, has an absorbance with respect to radiation of a second wavelength or a second wavelength range in the range of 600 nm to 50,000 nm of more than 50%.

9. Device (10, 10') according to one of the preceding claims,
wherein the device (10, 10') comprises an absorption area in the cavity (12) comprising or consisting of an absorbent material which, at least in a second temperature range, has an absorbance with respect to radiation of a second wavelength or a second wavelength range in the range of 600 nm to 50,000 nm of more than 50%, and/or
the device (10, 10') is selected from the group composed of syringes, application needles and compules, and/or
the device (10, 10') comprises an application tip (16) for applying the dental material (20), wherein the application tip (16) comprises an outlet opening for the dental material (20), wherein the outlet opening preferably has an outlet cross-sectional area in the range of 0.2 to 3.0 mm², preferably not more than 2.0 mm², and particularly preferably not more than 1.5 mm².

10. Method for treating a dental material (20) outside the human or animal body, with the following steps:
- providing (100, 200) a device (10, 10') according to one of Claims 1 to 9 comprising radiation-curable dental material (20) in the first temperature range,
- irradiating (110, 210) the provided device (10, 10') with radiation of a second wavelength or a second wavelength range, preferably with IR light of a wavelength in the range of 600 nm to 50,000 nm, in order to heat (130, 220) the dental material (20),
- reducing (140, 230) the viscosity of the dental material (20) by means of heating (130, 220) to a desired viscosity or less.

11. Method according to Claim 10, wherein, through the wall (14) of the provided device (10, 10'), before irradiation (110, 210) with the radiation of the second wavelength or the second wavelength range and during irradiation (110, 210) with the radiation of the second wavelength or the second wavelength range,
- radiation of the first wavelength or the first wavelength range is blocked and/or reflected, and
- radiation of the second wavelength or the second wavelength range is allowed to pass through to the dental material (20).

12. Method according to Claim 10 or 11, with the following further step:
- dispensing (150, 240) the dental material (20) from the device (10, 10').

13. Method according to Claim 12, with the following further step:
- irradiating (160, 250) the dental material (20) with radiation of the first wavelength or the first wavelength range so that the dental material (20) is cured.

14. Use of a filter material for producing a device (10, 10') according to Claim 2 or 3.

15. Dental system or kit, comprising
- a device (10, 10') according to one of Claims 1 to 9 and
- a radiation source (34) for the radiation of a second wavelength or a second wavelength range in the range of 600 nm to 50,000 nm.

## Revendications

1. Dispositif (10, 10') pour la réception et l'application de matériau dentaire (20), avec
une cavité (12) pour la réception du matériau dentaire (20) et
une paroi (14) entourant la cavité (12),
dans lequel la paroi (14) est imperméable, dans au moins une première plage de températures, au rayonnement d'au moins une première longueur d'onde ou d'une première gamme de longueurs d'onde dans la gamme de 100 nm à 500 nm, et
dans lequel la paroi (14) présente au moins une zone avec un matériau de support, de préférence un matériau en polyamide et/ou un matériau en polybutylène téréphtalate, et un matériau filtrant, qui est prévu à l'extérieur sur le matériau de support, à l'intérieur sur le matériau de support et/ou entre au moins deux couches du matériau de support et/ou est intégré dans le matériau de support,
**caractérisé en ce que** le matériau filtrant est choisi dans le groupe constitué :
- des dérivés de pérylène, 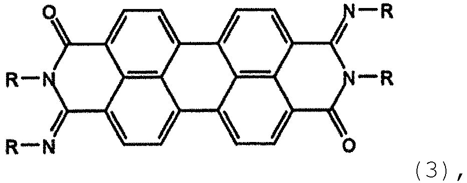 en particulier 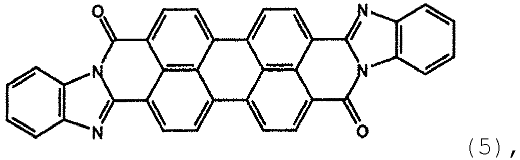 et 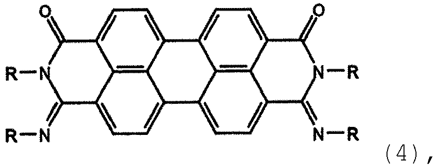 en particulier 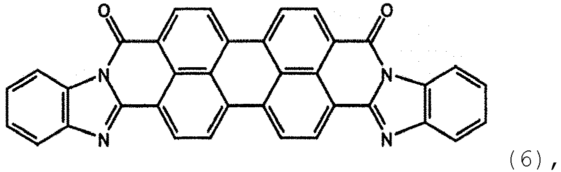 ainsi que des combinaisons de ceux-ci.

2. Dispositif (10, 10') selon la revendication 1, dans lequel la zone de la paroi (14) présente un matériau filtrant ou en est constitué,
dans lequel le matériau filtrant présente au moins dans la première plage de températures, de préférence au moins dans la première plage de températures et dans une deuxième plage de températures, pour le rayonnement de la première longueur d'onde ou de la première gamme de longueurs d'onde, un degré de transmission inférieur à 10 %, de préférence inférieur à 5 %,
dans lequel le matériau filtrant présente au moins dans la deuxième plage de températures pour le rayonnement d'une deuxième longueur d'onde ou d'une deuxième gamme de longueurs d'onde dans la gamme de 600 nm à 50 000 nm un degré de transmission supérieur à 50 %, de préférence supérieur à 65 %, de manière particulièrement préférée supérieur à 80 %.

3. Dispositif (10, 10') selon la revendication 2, dans lequel le matériau filtrant est choisi dans le groupe constitué de substances avec un squelette de pérylène, des composés diazoïques aromatiques et des combinaisons de ceux-ci.

4. Dispositif (10, 10') selon l'une quelconque des revendications précédentes,
dans lequel la zone de la paroi (14) présente un matériau thermochromique ou en est constituée, qui possède une température de commutation dans la plage de 25 à 50 °C,
dans lequel le matériau thermochromique, à une température au-dessous de la température de commutation, pour le rayonnement d'au moins une deuxième longueur d'onde ou d'une deuxième gamme de longueurs d'onde dans la gamme de 600 nm à 50 000 nm, possède un degré de transmission plus bas qu'à une température au-dessus de la température de commutation.

5. Dispositif (10, 10') selon la revendication 4, dans lequel le matériau thermochromique est choisi dans le groupe constitué de pigments anorganiques comprenant des sels métalliques ou oxydes métalliques, pour lesquels, du fait d'une transition de phase, d'une modification de la géométrie des ligands, d'une modification de la coordinence et/ou d'une modification du champ cristallin, un changement de couleur a lieu, de pigments organiques comprenant des cristaux liquides thermochromiques, de polymères conjugués et de leuco-colorants et des combinaisons de ceux-ci.

6. Dispositif (10, 10') selon l'une quelconque des revendications précédentes,
dans lequel la paroi (14) présente au moins un matériau de support, de préférence un matériau en polyamide et/ou un matériau en polybutylène téréphtalate, dans lequel le matériau filtrant et/ou le matériau thermochromique (14'') est prévu à l'extérieur sur le matériau de support (14'), à l'intérieur sur le matériau de support et/ou entre au moins deux couches du matériau de support et/ou est intégré dans le matériau de support, et/ou
dans lequel le quotient du degré de transmission de la zone de la paroi (14) au moins dans une deuxième plage de températures pour le rayonnement d'une deuxième longueur d'onde ou d'une deuxième gamme de longueurs d'onde dans la gamme de 600 nm à 50 000 nm et du degré de transmission de la paroi (14) dans au moins la première plage de températures pour le rayonnement au moins de la première longueur d'onde ou de la première gamme de longueurs d'onde est supérieur à 5, de préférence supérieur à 10, de manière particulièrement préférée supérieur à 50, et/ou
dans lequel la paroi (14) présente une zone de passage, qui est perméable au moins dans une deuxième plage de températures au rayonnement de la deuxième longueur d'onde ou de la deuxième gamme de longueurs d'onde, dans lequel la paroi (14) présente en outre sur une face opposée à la zone de passage une zone de réflexion, qui est conçue au moins dans la deuxième plage de températures pour réfléchir ou pour rétrodiffuser le rayonnement, traversant la cavité (12), de la deuxième longueur d'onde ou de la deuxième gamme de longueurs d'onde avec un degré de réflexion d'au moins 50 % dans la cavité (12), et/ou
dans lequel la première plage de températures est une plage de 15 à 25 °C et la deuxième plage de températures est une plage de 25 à 75 °C.

7. Dispositif (10, 10') selon l'une quelconque des revendications précédentes, comprenant dans la cavité (12) un matériau dentaire (20) durcissable par rayonnement,
dans lequel le matériau dentaire (20) est à durcir avec le rayonnement de la première longueur d'onde ou de la première gamme de longueurs d'onde, dans lequel le matériau dentaire (20) possède dans la première plage de températures, en particulier à une température de 20 °C, une viscosité supérieure à 400 Pa·s et dans lequel le matériau dentaire (20) possède dans une deuxième plage de températures, en particulier à une température de 60 °C, une viscosité inférieure à 150 Pa·s et/ou
dans lequel le matériau dentaire (20) durcissable par rayonnement est une composition composite à un composant comprenant
(A) des monomères,
(B) des charges et
(C) des amorceurs.

8. Dispositif (10, 10') selon la revendication 7, dans lequel le matériau dentaire (20) présente un composant absorbeur, qui possède au moins dans la deuxième plage de températures un degré d'absorption pour le rayonnement d'une deuxième longueur d'onde ou d'une deuxième gamme de longueurs d'onde dans la gamme de 600 nm à 50 000 nm supérieur à 50 %.

9. Dispositif (10, 10') selon l'une quelconque des revendications précédentes,
dans lequel le dispositif (10, 10') présente dans la cavité (12) une zone d'absorption, qui présente un matériau d'absorption ou en est constitué, qui possède au moins dans une deuxième plage de températures un degré d'absorption pour le rayonnement d'une deuxième longueur d'onde ou d'une deuxième gamme de longueurs d'onde dans la gamme de 600 nm à 50 000 nm supérieur à 50 %, et/ou
le dispositif (10, 10') est choisi dans le groupe constitué de seringues, canules d'application et compules et/ou
le dispositif (10, 10') présente une pointe d'application (16) pour l'application du matériau dentaire (20), dans lequel la pointe d'application (16) possède une ouverture de sortie pour le matériau dentaire (20), dans lequel l'ouverture de sortie possède de préférence une surface de section transversale de sortie dans la plage de 0,2 à 3,0 mm², de préférence non supérieure à 2,0 mm², de manière particulièrement préférée non supérieure à 1,5 mm².

10. Procédé pour le traitement d'un matériau dentaire (20) à l'extérieur du corps humain ou animal, avec les étapes suivantes :
- la fourniture (100, 200) d'un dispositif (10, 10') selon l'une quelconque des revendications 1 à 9 comprenant un matériau dentaire (20) durcissable par rayonnement dans la première plage de températures,
- l'exposition (110, 210) du dispositif (10, 10') fourni au rayonnement d'une deuxième longueur d'onde ou d'une deuxième gamme de longueurs d'onde, de préférence à une lumière infrarouge d'une longueur d'onde dans la gamme de 600 nm à 50 000 nm, pour le chauffage (130, 220) du matériau dentaire (20),
- la réduction (140, 230) de la viscosité du matériau dentaire (20) par le chauffage (130, 220) jusqu'à une viscosité souhaitée ou moins.

11. Procédé selon la revendication 10, dans lequel, avant l'exposition (110, 210) au rayonnement de la deuxième longueur d'onde ou de la deuxième gamme de longueurs d'onde et pendant l'exposition (110, 210) au rayonnement de la deuxième longueur d'onde ou de la deuxième gamme de longueurs d'onde
- le rayonnement de la première longueur d'onde ou de la première gamme de longueurs d'onde est bloqué et/ou réfléchi et
- le rayonnement de la deuxième longueur d'onde ou de la deuxième gamme de longueurs d'onde vers le matériau dentaire (20) est laissé passer
par la paroi (14) du dispositif (10, 10') fourni.

12. Procédé selon la revendication 10 ou 11, avec l'autre étape suivante :
- la distribution (150, 240) du matériau dentaire (20) à partir du dispositif (10, 10').

13. Procédé selon la revendication 12, avec l'autre étape suivante :
- l'exposition (160, 250) du matériau dentaire (20) au rayonnement de la première longueur d'onde ou de la première gamme de longueurs d'onde, de sorte que le matériau dentaire (20) durcit.

14. Utilisation d'un matériau filtrant pour la fabrication d'un dispositif (10, 10') selon la revendication 2 ou 3.

15. Installation dentaire ou kit, comprenant
- un dispositif (10, 10') selon l'une quelconque des revendications 1 à 9 et
- une source de rayonnement (34) pour le rayonnement d'une deuxième longueur d'onde ou d'une deuxième gamme de longueurs d'onde dans la gamme de 600 nm à 50 000 nm.
